# EUROPÄISCHE PATENTSCHRIFT

(11) **EP 0 281 829 B1**
(45) Veröffentlichungstag und Bekanntmachung des Hinweises auf die Patenterteilung: **04.05.1994**
(21) Anmeldenummer: 88102554.8
(22) Anmeldetag: 22.02.1988
(51) Int. Cl.: C07C 49/80, C07C 245/06, G01N 21/75, C09K 19/06

(54) **Addukte aus Ketoverbindungen und Anionen von Oxasäuren, Verfahren zu deren Herstellung, deren Verwendung sowie Azoketoverbindungen.**
Adducts of ketonic compounds with the anions of oxygenated acids, process for their preparation, their use and azo ketonic compounds
Adducts de composés cétoniques avec les anions d'acides oxygénés, leur procédé de préparation, leur application et composés azo cétoniques

(30) Priorität: 13.03.1987 CH 939/87; 16.12.1987 CH 939/87; 30.09.1987 CH 3801/87; 16.12.1987 CH 3801/87
(43) Veröffentlichungstag der Anmeldung: 14.09.1988
(73) Patentinhaber: Willi Möller AG, CH-8050 Zürich (CH)
(72) Erfinder: Simon, Wilhelm, Prof. Dr., CH-8006 Zürich (CH); Ernö, Pretsch, CH-9548 Horlingen-Matzingen (CH)
(74) Vertreter: Blum, Rudolf Emil Ernst

(56) Entgegenhaltungen:
- DE-A- 1 920 176
- US-A- 3 968 168
- CHIMIA, Nr. 11, November 1987, Seiten 397-398; C. BEHRINGER et al.: "Carbonate-selective chromoionophores"
- FIRST BIOELECTROANALYTICAL SYMPOSIUM, Matrafüred, 1986, Seiten 173-187; W. SIMON et al.: "Sensors for ions and neutral species based on a selective substrate permeation through membranes"
- ANALYTICAL CHEMISTRY, Band 59, Nr. 1, Januar 1987, Seiten 144-150, American Chemical Society; M.E. MEYERHOFF et al.: "Role of trifluoroacetophenone solvents and quaternary ammonium salts in carbonate-selective liquid membrane electrodes"
- J. ORG. CHEM, Band 44, Nr. 9, 1979, Seiten 1577-1578, American Chemical Society; A. FINIELS et al.: "Correlation of the reactivity of ketones relative to different nucleophiles"
- CHEMICAL ABSTRACTS, Band 94, 1981, Seite 610, Zusammenfassung Nr. 93033s, Columbus, Ohio, US; H.G. KRAFT et al.: "Spectroscopic investigations of chelate complexes with dicarbonyl ligands"
- BULL. CHEM. SOC. JPN., Band 55, 1982, Seiten 641-642, The Chemical Society of Japan; F. OGURA et al.: "Bis (P-methoxyphenyl) selenoxide as a mild and selective oxidizing agent"
- ELEKTROKHIMIYA, Band 21, Oktober 1985, S. 1335-1339, A. A. Zhdanov University, Leningrad, SU; A. L. SMIRNOVA et al: "Investigation of film-type carbonate-selective membranes based on the neutral chelating agent, hexyl trifluoroacetyl benzoate"

## Beschreibung

Organische Verbindungen, beispielsweise Dicarbonsäurediamide, die mit Kationen lipophile Komplexe bilden, werden sehr häufig eingesetzt, um die Konzentration der entsprechenden Kationen zu bestimmen. Beispielsweise werden entsprechende ionenempfindliche Teile, wie zum Beispiel Membranen von ionenempfindlichen Elektroden, die als Komponente die mit den Kationen Komplexe bilden, organische Verbindungen enthalten, verwendet, um die Konzentration der entsprechenden Kationen zu bestimmen. Es sind ferner Testvorrichtungen bekannt, welche eine entsprechende kationselektive Komponente auf einem Trägermaterial, zusammen mit einem Indikator, enthalten, beispielsweise einem pH-Indikator, wobei bei Anwesenheit der zu bestimmenden Kationen eine Veränderung einer Eigenschaft des Indikators feststellbar ist, beispielsweise eine Farbveränderung.

Im Gegensatz dazu sind im Stande der Technik kaum irgendwelche organischen Verbindungen beschrieben, die mit Anionen in Wechselwirkung treten und die als ionenempfindliche Komponente zu Bestimmung der Konzentration von Anionen herangezogen werden können.

Ziel der vorliegenden Erfindung war es, aus bestimmten Anionen und organischen Verbindungen Addukte herzustellen, beziehungsweise die entsprechenden adduktbildenden organischen Verbindungen zum Nachweis der entsprechenden Anionen heranzuziehen.

### BESCHREIBUNG DES STANDES DER TECHNIK

Zur Bestimmung der Konzentration von Anionen wurden bisher praktisch nur lipophile Ammoniumverbindungen als ionenempfindliche Komponente eingesetzt.

In der USA Patentschrift 3 429 785 werden Elektroden beschrieben, die in einer flüssigen, im wesentlichen wasserunmischbaren Phase ein quaternäres Salz eines aliphatischen Amines enthalten.

In der USA Patentschrift 3 723 281 werden gegenüber Bicarbonationen empfindliche Elektroden beschrieben, in denen die ionenempfindliche Membran als ionenempfindliche Komponente ein quaternäres Ammoniumsalz mit hohem Molekulargewicht, gelöst in einem Lösungsmittelsystem, enthält, das eine organische Verbindung und einen aliphatischen Alkohol mit 6 - 12 Kohlenstoffatomen enthält. Als organische Verbindung sind neben fluorierten Alkoholen und Estern auch Trifluoracetylbenzole genannt, in denen der Benzolkern ein oder zwei Alkylgruppen als Substituenten trägt. Es wurde in dieser USA Patentschrift angenommen, dass die ionenempfindliche Komponente der Membranen das quaternäre Amin ist, während die genannten organischen Verbindungen lediglich als Modifikatoren wirken.

Weiterentwicklungen dieser Elektroden, welche zur Bestimmung Von Kohlendioxid im Blutserum und Blutplasma eingesetzt werden konnten, sind in den Veröffentlichungen von Greenberg, J.A. Meyerhoff, M.E. in Anal. Chim. Acta 1982, 141, 57-64, sowie Scott et al. in Clin. Chem. (Winston-Salem, N.C.) 1986, 32, 137-41 und Smirnova, A.L. et al. in Electrokimiya 1985, 21, 1221-1224, beschrieben.

In allerjüngster Zeit zeigte es sich, dass das Trifluoracetyl-p-butylbenzol, das als Lösungsmittelkomponente der carbonatselektiven Elektroden der USA Patentschrift 3 723 281 bereits genannt ist, die Fähigkeit besitzt, mit dem Carbonatanion Addukte zu bilden, und zwar sowohl Addukte aus einem Mol Carbonatanion und einem Mol fluoriertem Keton als auch Addukte aus einem Mol Carbonatanion und zwei Molen fluoriertem Keton. Es sei in diesem Zusammenhang auf die Veröffentlichung von Meyerhoff M.E., Pretsch E., Welti D.H. und Simon W. in Analytical Chemistry, 1987, 59, Seiten 144 bis 150 verwiesen.

In dieser Veröffentlichung von M.E. Meyerhoff et al. wurden als organische Verbindung, die mit den Carbonatanionen ein Addukt bildet, ein einziges aromatisches Keton untersucht, nämlich das Trifluoracetyl-p-butylbenzol der folgenden Formel A 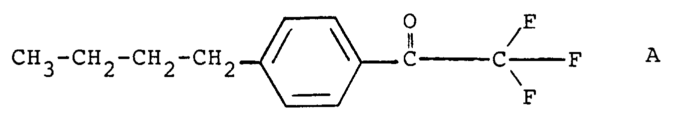
Des weiteren wurde in dieser Veröffentlichung als Carbonatsalz, das mit diesem aromatischen Keton ein Addukt bildet, hauptsächlich ein solches Salz verwendet, dessen Kation stark lipophile Eigenschaften aufweist, nämlich das Tridodecyl-methyl-ammoniumcarbonat der Formel, 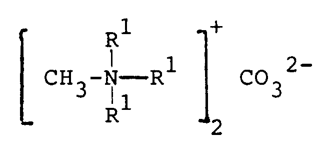
in welchem sämtliche drei Reste R¹ die Bedeutung von n-Dodecylresten aufweisen. In dieser Veröffentlichung sind jedoch auch lipophile Membranen beschrieben, die das Trifluoracetyl-p-butylbenzol in einer weichmacherenthaltenden Polyvinylchloridmembran enthalten, wobei die entsprechenden Membranen gegebenenfalls auch noch ein Chlorid mit lipophilem Kation, nämlich Tridodecylmethyl-ammoniumchlorid als weitere Komponente enthalten.

Carbonatselektive Membranen sind ferner in der Veröffentlichung von Smirnova, A.L.; Grekovich, A.L.; Materova, E.A. Electrokhimiya 1985, 21, 1335-1339 beschrieben. Die dort beschriebenen Membranen enthalten als Polymerkomponente ein weichmacherenthaltendes Polyvinylchlorid und ferner einen Ionenaustauscher, nämlich Tetradecylammoniumcarbonat und ausserdem Hexyl-trifluoracetylbenzoat als carbonatselektive Komponente, nämlich eine Verbindung, welche der oben angegebenen Formel A entspricht, wobei jedoch der Substituent des Benzolkernes nicht ein n-Butylrest ist, sondern eine mit Hexanol veresterte Carboxylgruppe. Wässrige Lösungen, die Ammoniumhydrogencarbonat, beziehungsweise Kaliumhydrogencarbonat enthielten, wurden mit Hilfe der Membranen untersucht.

In einer Veröffentlichung im J. Org. Chem. Vol. 44 Nr. 9, 1979, Seiten 1577 - 1578, wurde die Reaktionsfähigkeit verschiedener Monoketone gegenüber nucleophilen Reagenzien untersucht, darunter auch die Reaktivität gegenüber den Anionen von zwei Oxasäuren, nämlich Sulfitanionen und Chromatanionen. Bei den untersuchten 26 Ketonen handelt es sich um Monoketone, bei welchen sich die Ketogruppe in einem cycloaliphatischen Ring befindet. In keinem der genannten Ketone befindet sich jedoch, in Nachbarstellung zu der Ketogruppe ein Kohlenstoffatom, an das mindestens ein stark Elektronen anziehender Substituent gebunden ist.

Ketoverbindungen, bei denen an das Kohlenstoffatom in Nachbarstellung zu der Ketogruppe mindestens ein stark Elektronen anziehender Substituent gebunden ist, sind bereits in der Literatur beschrieben. Beispielsweise werden in der USA Patentschrift Nr. 3 968 168 1,1-Diphenylalkane beschrieben, die als Insektizide wirksam sind. Zu deren Herstellung wurden monophenylsubstituierte Ketone herangezogen, beispielsweise auch solche, bei denen an das Kohlenstoffatom in Nachbarstellung zu der Ketogruppe 1 - 3 Chloratome gebunden sind.

Gemäss der deutschen Offenlegungsschrift 1 920 176 können bromsubstituierte Phenone als Ausgangsmaterial bei der Herstellung von Hydroxycrotonsäurelaktonen eingesetzt werden.

Es wurde jetzt überraschenderweise gefunden, dass nicht nur Carbonatanionen und Bicarbonatanionen, sondern ganz allgemein die Anionen von organischen oder anorganischen Oxasäuren zur Adduktbildung mit Ketonen geeignet sind, bei denen die Ketogruppe an ihrer einen Seite an einen aromatischen oder heteroaromatischen Rest gebunden ist und an ihrer anderen Seite an ein Kohlenstoffatom, das mindestens einen stark elektronenanziehenden Substituenten trägt, der aus der Gruppe umfassend Fluoratome, Chloratome, Bromatome oder Nitrogruppen ausgewählt ist.

### BESCHREIBUNG DER ERFINDUNG

Ein Gegenstand der vorliegenden Erfindung sind Addukte aus Anionen und Ketoverbindungen, die dadurch gekennzeichnet sind, dass die Anionen solche von Oxasäuren sind und dass die Ketoverbindungen Monoketone der Formel I 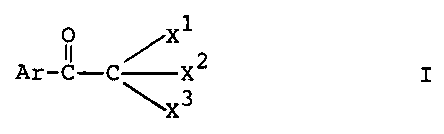
sind, in welcher
Ar ein substituierter oder unsubstituierter, ein- oder mehrkerniger, aromatischer oder ein substituierter oder unsubstituierter, ein- oder mehrkerniger, heterocyclischer Rest aromatischen Charakters ist, vorzugsweise ein Benzolkern, ein Naphthalin, ein Anthracen, ein Thiophen, ein Furan, ein Pyrrol, ein Indol, ein Benzofuran, ein Benzothiophen, ein Pyridin, ein Chinolin, ein Pyrazin oder ein Triazin ist, wobei die aromatischen oder heteroaromatischen Ringsysteme als Substituenten eine oder mehrere der folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierge Cycloalkylgruppen und substituierte oder unsubstituierte Arylgruppen tragen können und wobei
X¹, X² und X³ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und unter der weiteren Voraussetzung, dass dann, wenn Ar Alkoxycarbonylphenyl oder Alkylphenyl ist, nicht sämtliche Reste X¹,X² und X³ die Bedeutung von Fluoratomen haben dürfen.

Wie bereits erwähnt wurde, sind aus der Veröffentlichung Analytical Chemistry, Vol. 59, Seiten 144-150, 1967, und aus der Veröffentlichung Smirnova, A.L.; Grekovich, A.L.; Materova, E.A. Electrokhimiya 1985, 21, 1335-1339 bereits solche Addukte bekannt geworden, in denen das Anion ein Carbonatanion oder Bicarbonatanion ist und die adduktbildende Ketoverbindung der Formel I entspricht, wobei Ar ein alkylsubstituiertes Phenyl oder ein mit einer Alkoxycarbonylgruppe substituiertes Phenyl ist und in denen ferner sämtliche Substituenten X¹, X² und X³ Fluoratome bedeuten. Diese aus dem Stande der Technik bekannten Addukte wurden durch den obigen Disclaimer aus dem Schutzbereich ausgeklammert.

Bevorzugte Anionen von Oxasäuren, welche mit den Monoketonen der Formel I die erfindungsgemässen Addukte bilden, sind CO₃⁻⁻, HCO₃⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, JO₃⁻, NO₃⁻, NO₂⁻, CrO₄⁻⁻, HCrO₄⁻, Cr₂O₇⁻⁻, HCr₂O₇ oder die Anionen von Carbonsäuren, Hydroxycarbonsäuren, Ketocarbonsäuren, Aminocarbonsäuren oder die Anionen von Peptiden, oder Anionen von organischen Derivaten der Phosphorsäure, insbesondere Estern der Phosphorsäure, der Diphosphorsäure oder Triphosphorsäure, beispielsweise der Adenylsäure, des Adenosindiphosphats oder Adenosintriphosphats.

Speziell bevorzugte erfindungsgemässe Addukte sind diejenigen, in welchen die Ketoverbindungen der Formel I solche sind, in welchen die Ketogruppe ein Teil eines Chromophoren ist oder die Ketoverbindungen eine fluoreszierende Gruppe aufweisen, wobei sich diese Ketoverbindungen von dem Addukt derselben mit einem Anion einer Oxasäure bezüglich der Absorption im sichtbaren Bereich oder im ultravioletten Bereich unterscheiden, oder wobei durch die Adduktbildung der Ketoverbindung mit dem Anion der Oxasäure eine Fluoreszenz hervorgerufen oder eine Fluoreszenz gelöscht wird.

Wie aus der Formel I ersichtlich ist, steht in den Ketoverbindungen, welche die erfindungsgemässen Addukte bilden, die Ketogruppe in Konjugation zu dem aromatischen oder heteroaromatischen Rest Ar. Bei der Bildung des Adduktes aus diesen Ketoverbindungen der Formel I mit den Anionen der Oxasäuren, wird diese Konjugation aufgehoben, und dementsprechend tritt im allgemeinen bei der Bildung des Adduktes aus der Oxasäure und der Ketoverbindung der Formel I eine Verschiebung der Absorption im sichtbaren Bereich, bzw. im ultravioletten Bereich in Richtung zu kürzeren Wellenlängen auf, also eine hypsochrome Verschiebung, verglichen mit der Absorption der entsprechenden Ketoverbindung der Formel I.

Bevorzugte Addukte aus Anionen von Oxasäuren und solchen Ketokomponenten, in welchen die Ketogruppe ein Teil eines Chromophoren ist, und in welcher die entsprechenden Ketoverbindungen im Vergleich zu den Addukten derselben mit Anionen der Oxasäuren eine Verschiebung der Absorption im sichtbaren Bereich oder im ultravioletten Bereich aufweisen, sind solche mit Azoketoverbindungen, welche die folgende Formel II 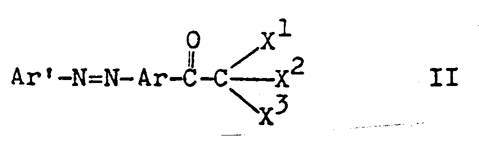
aufweisen, in welcher die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und
X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist.

Diese Azoketoverbindungen, welche die Fähigkeit haben, mit den Anionen von Oxasäuren Addukte zu bilden, sind bisher in der Literatur noch nicht beschrieben worden.

Ein weiterer Gegenstand der vorliegenden Erfindung sind daher diese neuen Azoketoverbindungen der Formel II, 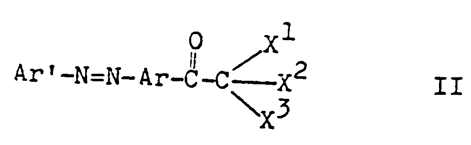
in welchen die Gruppen
Ar und Ar', sowie X¹, X² und X³ diejenige Bedeutung besitzen, die im Zusammenhang mit Addukten dieser Azoketoverbindungen der Formel II und Anionen von Oxasäuren oben erläutert wurde .

Zu den Azoketoverbindungen der Formel II gehören unter anderem die Azoketoverbindungen, welche die folgende Formel XIII 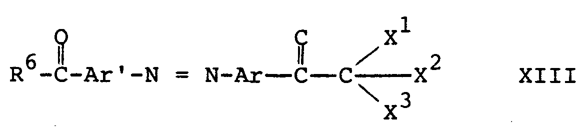
aufweisen, in welcher Ar und Ar', sowie X¹, X² und X³ die im Zusammenhang mit Formel II angegebene Bedeutung besitzen und
R⁶ einen organischen Rest darstellt, vorzugsweise einen solchen, der in Nachbarstellung zu der Carbonylgruppe mindestens einen stark elektronenanziehenden Substituenten trägt, sodass bevorzugt der Rest R⁶ eine Gruppe der Formel 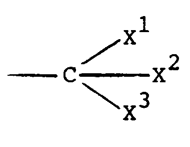
ist, in welcher X¹, X² und X³ die im Zusammenhang mit Formel II angegebene Bedeutung besitzen.

In bevorzugten Azoketoverbindungen der oben angegebenen Formel XIII sind sowohl der Rest Ar als auch der Rest Ar' Phenylreste, sodass diese Verbindungen dann beispielsweise die folgende Struktur: 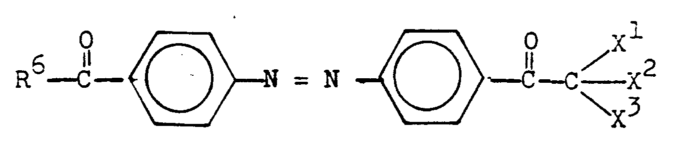
aufweisen, wobei allenfalls die Benzolkerne unabhängig voneinander noch Substituenten tragen können.

In den Addukten aus Oxasäuren und solchen Ketoverbindungen der Formel I, in welchen Ar ein Benzolkern ist, der als Substituenten ein oder mehrere Alkylgruppen oder eine Alkoxycarbonylgruppe trägt, hat die Gruppierung der Formel 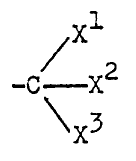
bevorzugt die Bedeutunge einer der folgenden Gruppierungen:
eine Monochlormethylgruppe, Dichlormethylgruppe, Trichlormethylgruppe, Monofluormethylgruppe, Difluormethylgruppe, Monochlormonofluormethylgruppe, Monochlordifluormethylgruppe, Dichlormonofluormethylgruppe oder ein Bromanaloges der genannten Chlorverbindungen, beziehungsweise Chlorfluorverbindungen.

Spezielle Beispiele für Verbindungen der Formel I, in welcher der Rest Ar ein Benzolkern ist, der gegebenenfalls mit ein oder mehr Alkylsubstituenten oder Halogenalkylsubstituenten substituiert ist, sind die folgenden Verbindungen:
Trifluoracetophenone, die gegebenenfalls im Benzolkern Halogenatome, oder Halogenalkylgruppen, z.B. Trifluormethylgruppen, als Substituenten tragen, Monochloracetylbenzole, Dichloracetylbenzole und Trichloracetylbenzole, die gegebenenfalls im Benzolkern Alkylgruppen oder Halogenalkylgruppen als Substituenten tragen, wie zum Beispiel Monochloracetyl-p-butylbenzol und die entsprechenden Dichloracetyl- und Trichloracetylverbindungen.

In den Azoverbindungen der Formel II und auch in den Azoverbindungen der Formel XIII bedeuten vorzugsweise die Reste Ar und Ar' unabhängig voneinander die aromatischen Ringe, beziehungsweise Ringsysteme, Benzol, Naphthalin oder Anthracen oder die heterocyclischen Reste aromatischen Charakters Thiophen, Furan, Pyrrol, Indol, Benzofuran, Benzothiophen, Pyridin, Chinolin, Pyrazin oder Triazin. Die entsprechenden aromatischen oder heteroaromatischen Ringe oder Ringsysteme können unsubstituiert sein oder ein oder mehrere Substituenten tragen, vorzugsweise solche, die aus der folgenden Gruppe ausgewählt sind: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen.

Bevorzugte Azoverbindungen der Formel II, bei denen eine Verschiebung der Absorption im sichtbaren Bereich oder im ultravioletten Bereich auftritt, wenn sie mit den Anionen von Oxasäuren in Wechselwirkung treten, sind diejenigen der folgenden Formel III 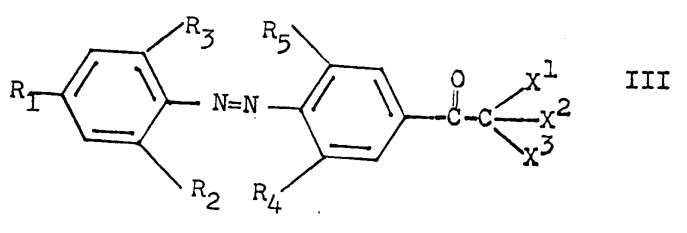
in welcher die Substituenten
R₁, R₂, R₃, R₄ und R₅ unabhängig voneinander die Bedeutung von Wasserstoffatomen, Hydroxygruppen, Alkyläthergruppen, Aryläthergruppen, veresterten Hydroxygruppen, Aminogruppen, Alkylaminogruppen, deren Alkylrest gegebenenfalls substituiert ist, Dialkylaminogruppen, deren Alkylgruppen gegebenenfalls substituiert sind, Arylaminogruppen, deren Arylrest gegebenenfalls substituiert ist, Nitrogruppen, gegebenenfalls substituierten Alkylgruppen, gegebenenfalls substituierten Alkenylgruppen, gegebenenfalls substituierten Alkinylgruppen oder substituierten oder unsubstituierten ein- oder mehrkernige aromatischen oder substituierten oder unsubstituierten ein- oder mehrkernigen heterocyclischen Resten aromatischen Charakters aufweisen.

Von diesen bevorzugten Azoverbindungen der Formel III sind wieder diejenigen speziell bevorzugt, in welchen die Reste R₄ und R₅ Wasserstoffatome sind und ein oder zwei der Reste R₁, R₂ und R₃ die Bedeutung einer Hydroxygruppe, einer verätherten Hydroxygruppe, einer veresterten Hydroxygruppe, einer Nitrogruppe, einer Aminogruppe, einer Monoalkylaminogruppe mit gegebenenfalls substituierter Alkylgruppe, einer Dialkylaminogruppe mit gegebenenfalls substituierter Alkylgruppe oder einer Aryl-, bzw. Diarylaminogruppe mit gegebenenfalls substituiertem Arylrest aufweist, während die übrigen der Reste R₁, R₂ und R₃ wiederum die Bedeutung von Wasserstoffatomen besitzen.

Von dieser bevorzugten Klasse der Azoverbindungen der Formel III sind wiederum diejenigen speziell bevorzugt, in welchen die Reste R₂, R₃, R₄ und R₅ die Bedeutung von Wasserstoffatomen aufweisen und der Rest
R₁ eine Hydroxygruppe, eine verätherte Hydroxygruppe, eine veresterte Hydroxygruppe, eine Nitrogruppe, eine Aminogruppe, eine Monoalkylaminogruppe, deren Alkylgruppe gegebenenfalls substituiert ist, eine Dialkylaminogruppe, deren Alkylgruppen gegebenenfalls substituiert sind, oder eine Arylaminogruppe, deren Arylrest gegebenenfalls substituiert ist, bedeutet.

Beispiele für diese zuletzt genannte Klasse an Ketoverbindungen der Formel III sind diejenigen, in welchen die Reste R₂, R₃, R₄ und R₅ Wasserstoffatome sind und der Rest
R₁ eine Hydroxygruppe, eine mit einem Alkylrest von 1 - 20 Kohlenstoffatomen, vorzugsweise einem Alkylrest mit 8 - 15 Kohlenstoffatomen, beispielsweise einem entsprechenden geradkettigen Alkylrest, verätherte Hydroxygruppe ist oder eine Hydroxygruppe bedeutet, die mit einer Alkancarbonsäure verestert ist, beispielsweise mit einer Alkancarbonsäure mit insgesamt 2 - 20 Kohlenstoffatomen, vorzugsweise einer Alkancarbonsäure mit 6 - 15 Kohlenstoffatomen, beispielsweise einer entsprechenden Alkancarbonsäure deren Alkylgruppe ein geradkettiger Alkylrest ist.

Ein weiteres Beispiel für bevorzugte Ketoverbindungen der Formel III, in welchen die Reste R₂, R₃, R₄ und R₅ die Bedeutung von Wasserstoffatomen haben, sind diejenigen, in welchen R₁ eine Alkylaminogruppe oder Dialkylaminogruppe ist, in der die Alkylreste gegebenenfalls Substituenten tragen. Speziell bevorzugt bedeutet der Rest R₁ eine Dialkylaminogruppe mit 2 - 12 Kohlenstoffatomen pro Alkylgruppe, wobei entsprechende längerkettige Dialkylaminogruppen gegebenenfalls in der Kette durch ein oder mehr Aethersauerstoffatome oder Estergruppierungen unterbrochen sind. Beispiele für die zuletzt genannte Klasse an Verbindungen sind diejenigen, in denen der Rest R₁ eine Di(alkanol)aminogruppe darstellt, wobei jede der beiden Hydroxygruppen des Di(alkanol)amines mit einer Carbonsäure verestert ist, beispielsweise einer Alkancarbonsäure mit 2 - 10 Kohlenstoffatomen.

Viele der Ketoverbindungen, der Formel I, welche die erfindungsgemässen Addukte mit den Anionen von Oxasäuren bilden (unter _{"}Addukte" sollen auch Chelate und Komplexe, mit einbezogen werden) sind in der Literatur bereits beschrieben. Sofern es neue Verbindungen sind, können sie nach chemischen Verfahren hergestellt werden, die in der Literatur bereits beschrieben sind. So können die entsprechenden Ketoverbindungen durch Oxydation von entsprechenden sekundären Alkoholen hergestellt werden.

Ketoverbindungen, in welchen die Ketogruppe an einen aromatischen oder heteroaromatischen Rest gebunden ist, können nach dem Prinzip der Friedel-Crafts-Acylierung durch Umsetzung der entsprechenden aromatischen, beziehungsweise heteroaromatischen Verbindung mit einem Acylhalogenid hergestellt werden. Der entsprechende Reaktionsmechanismus für die Ketoverbindungen der Formel I sei anhand des folgenden Reaktionsschemas veranschaulicht: 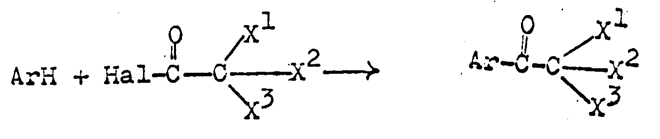
Derartige Acylierungen werden im allgemeinen in Anwesenheit einer Lewis-säure, beispielsweise Aluminiumchlorid, durchgeführt.

Azoketoverbindungen, beispielsweise die Verbindungen der weiter vorne angegebenen Formel XIII oder die Verbindungen der weiter vorne angegebenen Formel II können durch eine Azokupplung erzeugt werden.

Ein weiterer Gegenstand der Erfindung ist dementsprechend ein Verfahren zur Herstellung der Azoketoverbindungen der Formel II: 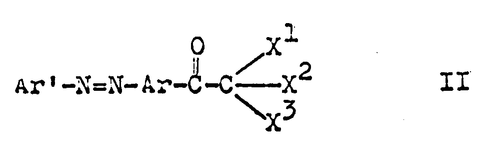
in welchen die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und
X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituenten X¹, X² und X³ ein stark Elektronen anziehender Substituent, ausgewählt aus der Gruppe Fluoratome, Chloratome, Bromatome oder Nitrogruppen ist. Diese Verbindungen werden hergestellt, indem man entweder ein primäres aromatisches oder heteroaromatisches Amin der Formel V 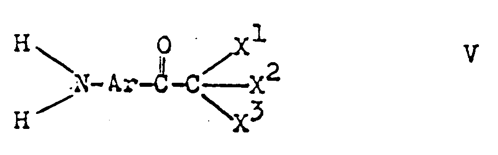
diazotiert und einer Azokupplung mit einer aromatischen oder heteroaromatischen Verbindung der Formel VI

Ar'-H VI

unterwirft, wobei sich die Azoverbindung der Formel II bildet,
oder indem man ein primäres aromatisches oder heteroaromatisches Amin der Formel VII 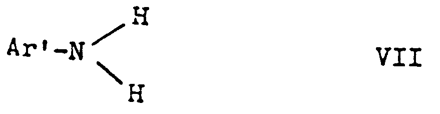
diazotiert und dieses einer Azokupplung mit einer aromatischen oder heteroaromatischen Verbindung der Formel VIII 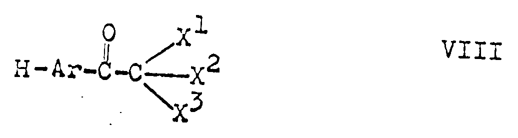
unterwirft, wobei sich die Azoverbindung der Formel II bildet.

Nach einem analogen Verfahren können auch die Ketoazoverbindungen der weiter vorne angegebenen Formel III hergestellt werden und das Prinzip dieser Herstellungsverfahren wird seit langem für die Herstellung von Azofarbstoffen eingesetzt.

Bei der Durchführung der oben veranschaulichten Azokupplung wird das primäre Amin der Formel V, beziehungsweise VII in allgemeinen durch Umsetzung mit einem Nitrit in saurem Medium in das entsprechende Diazoniumsalz über-geführt und dieses reagiert dann mit der entsprechenden aromatischen oder heteroaromatischen Verbindung der Formel VI, beziehungsweise VIII unter Bildung des gewünschten Endproduktes der Formel II.

Ein weiterer Gegenstand der Erfindung ist das Verfahren zur Herstellung der erfindungsgemässen Addukte aus Anionen von Oxasäuren und Ketoverbindungen. Dieses Verfahren ist dadurch gekennzeichnet, dass man das Anion einer Oxasäure mit der entsprechenden Ketoverbindung in Berührung bringt, wobei sich das Addukt bildet.

Ein weiterer Gegenstand der Erfindung ist die Verwendung der Ketoverbindungen der weiter vorne angegebenen Formel I zum Nachweis von Anionen von Oxasäuren, indem man die Ketoverbindungen der Formel I mit den nachzuweisenden Anionen in Berührung bringt, unter Bildung der erfindungsgemässen Addukte.

Bevorzugte Anionen von Oxasäuren, die unter Verwendung dieser Ketoverbindungen nachgewiesen werden können, sind die Anionen, welche bereits weiter vorne genannt wurden. Ebenso wurden auch die bei dieser Verwendung bevorzugt einzusetzenden Monoketoverbindungen und Azo-ketoverbindungen bereits weiter vorne näher erläutert.

Bei dieser Verwendung kann man die Ketoverbindung als ionenselektive Komponente eines ionenselektiven Teiles zur Bestimmung der Konzentration der Anionen der Oxasäuren einsetzen, beispielsweise als ionenselektive Komponente einer Membran einer ionenselektiven Elektrode zur Bestimmung der Konzentration der Anionen.

Bei der Verwendung der Ketoverbindungen zum Nachweis von Anionen von Oxasäuren in Probelösungen bildet sich die Additionsverbindung der entsprechenden Ketoverbindung mit dem nachzuweisenden Anion der Oxasäure dann, wenn der die Ketoverbindung enthaltende ionenselektive Teil, beispielsweise die Membran einer ionenselektiven Elektrode, mit dem Anion des Messgutes und/oder den Anionen der Elektrodenfüllung in Berührung bringt.

Die ionenselektiven Membranen entsprechender gegenüber den Anionen von Oxasäuren empfindlicher Elektroden können gegebenenfalls als weitere Komponente lipophile quaternäre Ammoniumionen enthalten, um eine Störung der Messwerte von Kationen, also eine sogenannte Kationeninterferenz zu vermeiden. Als Beispiel für lipophile Ammoniumverbindungen seien die Salze von Ammoniumverbindungen genannt, die mindestens eine langkettige aliphatische Gruppe aufweisen, wie zum Beispiel Tridodecyl-methylammoniumchlorid. Die ionenselektiven Membranen, die als Liganden die Carbonylverbindung enthalten, welche mit den Anionen von Oxasäuren in Wechselwirkung tritt, können unter Verwendung von Polyvinylchlorid, eines Weichmachers (beispielsweise Bis(2-aethylhexyl)sebacat oder o-Nitrophenyl-octyläther) und allenfalls eines lipophilen Ammoniumsalzes in der Weise hergestellt werden, die in der bereits früher genannten Veröffentlichung von M.E. Meyerhoff, Analytical Chemistry, 1987, 59, 144, beschrieben ist.

Gemäss einer bevorzugten Ausführungsart der Verwendung der Ketoverbindungen zum Nachweis von Anionen von Oxasäuren werden als Farbindikatoren solche Ketoverbindungen verwendet, in welchen die Ketogruppe ein Teil eines Chromophoren ist, oder solche Ketoverbindungen, die eine fluoreszierende Gruppe aufweisen. Es tritt dann durch die Reaktion des Anions der Oxasäuren mit der Ketoverbindung eine Verschiebung der Absorption der Ketoverbindung im sichtbaren Bereich oder im Ultraviolettbereich auf oder es wird eine Fluoreszenz hervorgerufen oder eine Fluoreszenz gelöscht.

Diese als Farbindikatoren wirkenden Carbonylverbindungen können als Komponente einer Testvorrichtung verwendet werden, wobei die Carbonylverbindung sich auf einem, bzw. in einem Trägermaterial befindet, beispielsweise einem Kunststoffträgermaterial oder einem Trägermaterial aus Papier, oder wobei die Testvorrichtung ein Sensor ist, der die Carbonylverbindung enthält, und der bei Berührung mit Anionen von Oxasäuren eine Farbveränderung im sichtbaren Bereich oder im ultravioletten Bereich oder ein Auftreten einer Fluoreszenz oder ein Löschen einer Fluoreszenz zeigt.

Die entsprechenden Sensoren sind beispielsweise Sensoren von Optoden, bei denen der Sensor in schwer zugängliche Bereiche eingeführt werden kann und die Aenderung der Farbe oder Fluoreszenz bei Kontakt mit den Anionen der Oxasäuren über einen Transmitter, beispielsweise einen Lichtleiter, beobachtet wird.

Bevorzugte Ketoverbindungen, die als Farbindikatoren, beziehungsweise als Komponente von Testvorrichtungen, wie Sensoren, eingesetzt werden, sind diejenigen Monoketoverbindungen der Formel I, in welchen die entsprechende Ketogruppe oder mindestens eine der Ketogruppen, einen Teil eines Chromophoren darstellen oder die entsprechenden Ketoverbindungen, die eine Gruppierung aufweisen, die zu einer Fluoreszenz befähigt ist.

Speziell bevorzugte Ketoverbindungen, die als Farbindikatoren, beziehungsweise als Komponente einer Testvorrichtung, beispielsweise eines Sensors, verwendet werden, sind die Azoketoverbindungen der weiter vorne angegebenen Formel II, bzw. die bevorzugten Azoketoverbindungen der weiter vorne angegebenen Formeln III und XIII.

Entsprechende Farbindikatoren, einschliesslich der fluoreszierende Gruppen aufweisenden Indikatoren, können direkt der Messlösung zugesetzt werden, wobei eine Farbveränderung im sichtbaren Bereich oder ultravioletten Bereich oder eine Ausbildung oder Löschung der Fluoreszenz der Anionen der Oxasäuren qualitativ oder halb quantitativ in der Testlösung bestimmt werden können. Bei der Durchführung von quantitativen Bestimmungen kann zu der den Indikator enthaltenden Messlösung titrimetrisch eine Lösung einer bekannten Konzentration des zu bestimmenden Anions zugesetzt und der Umschlagspunkt bestimmt werden.

Entsprechende Testvorrichtungen, welche die Farbindikatoren, beziehungsweise Fluoreszenzindikatoren auf oder in einem Trägermaterial, beispielsweise Kunststoff oder einem feuchtigkeitsaufnehmenden Material, wie Papier, enthalten, können beispielsweise die Form von Teststreifen aufweisen, die dann bei der Bestimmung der Anionen von Oxasäuren in die Probeflüssigkeit eingetaucht werden.

Wenn entsprechende Testvorrichtungen oder Sensoren mit den Anionen von Oxasäuren in Berührung kommen, beispielsweise in eine Flüssigkeit eingetaucht werden, die derartige Anionen enthält, ist eine Farbveränderung im sichtbaren Bereich oder im ultravioletten Bereich oder eine Löschung einer Fluoreszenz oder das Auftreten einer Fluoreszenz feststellbar. Auch hier ist durch die Stärke der Farbveränderung, beziehungsweise die Stärke der Aenderung der Fluoreszenz, die quantitative oder halbquantitative Bestimmung der Konzentration der Anionen der Oxasäuren in der Probelösung möglich.

Gemäss einer bevorzugten Ausführungsart der Erfindung sind die Testvorrichtungen die Sensoren von sogenannten Optoden, die in schwer zugängliche Bereiche eingeführt werden, wobei die Farbveränderung an dem Sensor im sichtbaren Lichtbereich oder im ultravioletten Lichtbereich oder eine Aenderung der Fluoreszenz des Sensors mit Hilfe eines Transmitters, beispielsweise eines Lichtleiters, beobachtet wird. Derartige Optoden können beispielsweise im klinischen Bereich eingesetzt werden, um dann an schwer zugänglichen Stellen, beispielsweise im Magen-Darm-trakt, in Venen oder Arterien, eine Bestimmung der Konzentration von Oxasäuren durchzuführen.

Bevorzugte Anionen von Oxasäuren, zu deren Bestimmung die Ketoverbindungen herangezogen werden können, sind bereits weiter vorne genannt. Zu den speziell bevorzugten Anionen gehören Carbonatanionen und Bicarbonatanionen. Dementsprechend können die Sensoren der oben erwähnten Optoden beispielsweise zur Bestimmung der Konzentration des Kohlendioxides im Blut herangezogen werden.

Die Ketoverbindungen, welche die erfindungsgemässen Addukte mit den Anionen von Oxasäuren bilden können auch, als Flüssigkristalle verwendet werden. In diesem Anwendungsbereich sind solche Ketoverbindungen bevorzugt, bei denen grössere Teilbereiche des Moleküles in der Lage sind, eine planare Struktur auszubilden, wie zum Beispiel die entsprechenden Azoverbindungen der Formeln XIII und II.

Die Erfindung sei nun anhand von Beispielen näher erläutert:

### Beispiel 1

Es wurden Monoketone der folgenden Formel 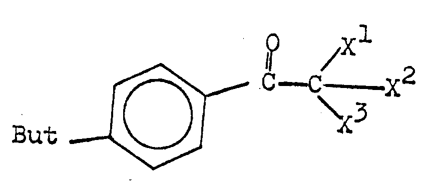
untersucht, in welcher But eine n-Butylgruppe darstellt.

Monochloracetyl-p-butylbenzol, abgekürzt als MCABB,
Dichloracetyl-p-butylbenzol, abgekürzt als DCABB,
Trichloracetyl-p-butylbenzol, abgekürzt als TCABB.

Alle drei untersuchten Ketone waren in der Lage, mit Carbonatanionen Addukte zu bilden. Sowohl anhand des Ultraviolett-Absorptionsspektrums als auch anhand des kernmagnetischen Resonanzspektrums, also des ¹³CNMR, konnte gezeigt werden, dass durch die Adduktbildung mit den Carbonatanionen die Carbonylgruppe der Monoketone verschwand.

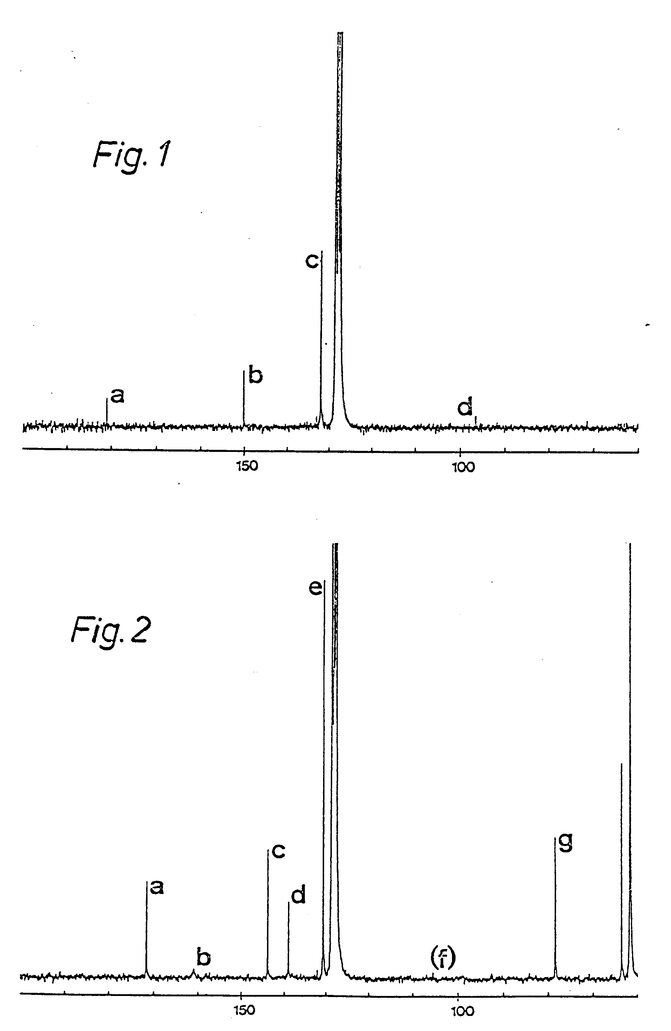

In Figur 1 ist das kernmagnetische Resonanzspektrum des Trichloracetyl-p-butylbenzols dargestellt. In Fig. 2 ist das kernmagnetische Resonanzspektrum des Adduktes der Carbonationen an das Trichloracetyl-p-butylbenzol veranschaulicht. Man sieht, dass in diesem Spektrum bei 78,5 ppm ein neues Signal zu finden ist, das der durch die Adduktbildung aus der Carbonylgruppe entstandenen Gruppierung entspricht.

Ferner zeigt ein Vergleich der Fig. 1 mit der Fig. 2 die chemische Verschiebung der jeweiligen Signale.

### Beispiel 2

Die in Beispiel 1 beschriebenen Monoketone, wurden mit Perchlorat-anionen, beziehungsweise Phosphatanionen, bzw. Sulfatanionen, zusammengebracht. Auch in diesen Fällen zeigte der Vergleich der UV Spektren und der Spektren im sichtbaren Bereich signifikante Aenderungen im Absorptionsverhalten.

Die in den nachfolgenden Beispielen 3 bis 7 beschriebenen Azoketoverbindungen wiesen die folgende Formel IIIa 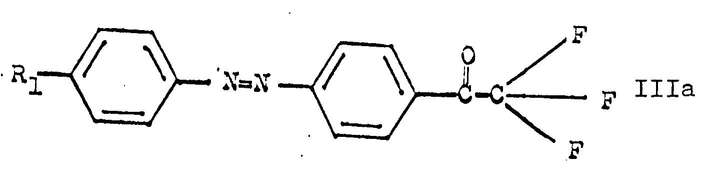
auf.

### Beispiel 3

### Herstellung von 4-Trifluoracetyl-4'-hydroxy-azobenzol

Die im Titel genannte Verbindung entspricht der oben angegebenen Formel IIIa, wobei R₁ eine Hydroxygruppe ist.

Als Ausgangsmaterial wurde das p-Trifluoracetyl-anilin verwendet, das in der Veröffentlichung von K.J. Klabunde und Dr. D.J. Burton, im J. Org. Chem. 35 (1970), Seite 1711 und ff beschrieben ist.

Eine Suspension von 2,84 g (15,00 mmol) an den p-Trifluoracetyl-anilin wurde in 45 ml einnormaler Salzsäure bei 25° C während einer Stunde gerührt, bis die gesamte Aminoverbindung in Lösung gegangen war.

Die blassgelbe Lösung wurde filtriert, in einem Eisbad auf 5° C gekühlt und dann setzte man 1,03 g (15,000 mmol) an Natriumnitrit zu. Es bildete sich dabei eine gelbe Lösung des Diazoniumsalzes.

1,41 g (15,00 mmol) an Phenol wurden in 60 ml einer ein-normalen Natriumacetatlösung gelöst. In diese Lösung wurde die gelbe Lösung des Diazoniumsalzes eingegossen. Innerhalb von weniger als 5 Min. fielen orange gefärbte Kristalle aus und diese wurden dann nach zwei Stunden abfiltriert. Die Kristalle wurden mit 200 ml Wasser gewaschen und dann zwei Tage bei 40° C getrocknet.

Man erhielt so 3,90 g (13,25 mmol) der im Titel genannten Verbindung in Form von roten Nadeln, welche einen Schmelzpunkt von 132 - 133° C aufwiesen. Die Ausbeute entsprach 88 % der theoretischen Ausbeute.

In Infrarotspektrum, aufgenommen in Chloroform, zeigte dieses Produkt eine Absorption bei 1715 cm⁻¹, entsprechend der Gruppierung der Formel -COCF₃.

Das kernmagnetische Resonanzspektrum, ¹H-NMR(300 MHz, CDCl₃), ergab 10,50 (s, 1H, OH); 8,20-8,23 (m, 2H); 7,90-8,02 (m, 4H); 6,98-7,01 (m, 2H).

Im Massenspektrum ergab das Produkt M⁺ m/e 294.

Die Elementaranalyse dieses Produktes der Summenformel C₁₄H₉N₂O₂F₃ ergab:

| | | | |
|---|---|---|---|
| berechnet: | C = 57,15; | H = 3,08; | N = 9,52 % |
| gefunden: | C = 57,00; | H = 2,91; | N = 9,28 % |

### Beispiel 4

### Herstellung von 4-Trifluoracetyl-4'-dodecyloxy-azobenzol

Die im Titel genannte Verbindung entspricht der Formel IIIa, wobei der Rest R₁ eine Alkyläthergruppierung der Formel

-O-(CH₂)₁₁-CH₃

ist.

Eine Mischung von 0,50 g (1,70 mmol) des nach dem Verfahren gemäss Beispiel 3 hergestellten 4-Trifluoracetyl-4'-hydroxy-azobenzoles, 15 g (80,5 mmol) an Dodecanol und 10 Tropfen konzentrierter Schwefelsäure (2,5 mmol) wurde 12 Stunden lang auf 100° C erhitzt.

Die Dünnschichtchromatographie (Laufmittel Diäthyläther) zeigte, dass eine geringe Menge des Ausgangsmaterials unumgesetzt zurückblieb.

Die gekühlte Mischung wurde zweimal gereinigt, indem man eine Chromatographie auf einer Säule, die mit 60 g 230-400 mesh SiO₂ (Fluka AG, Schweiz) gefüllt war, durchführte. Als Elutionsmittel verwendete man eine Mischung aus einem Teil Dichlormethan und vier Teilen Hexan.

Man erhielt so 0,42 g (0,91 mmol) des im Titel genannten Produktes in Form von roten Nadeln. Die Ausbeute betrug 53 % der theoretischen Ausbeute und dieses nahezu reine Produkt besass einen Schmelzpunkt von 72 - 73,5° C.

Dieses nahezu reine Produkt wurde aus Diäthyläther umkristallisiert und man erhielt so 0,35 g des gereinigten Produktes, dessen Schmelzpunkt 73 - 74° C betrug.

Im Infrarotspektrum, aufgenommen in Chloroform, zeigte dieses Produkt eine Absorption bei 1715 cm⁻¹, entsprechend der Gruppe der Formel

-COCF₃.

Das kernmagnetische Resonanzspektrum,
¹H-NMR (300 MHz, CDCl₃) ergab
8,19-8,22 (m, 2H); 7,93-7,99 (m, 4H); 6,99-7,04 (m, 2H); 4,06 (t, 2H); 1,83 )m, 2H); 1,36-1,46 (m, 18H); 0,88 (t, 3H).

Das Massenspektrum dieses Produktes lieferte M⁺ m/e 462.

Die Elementaranalyse dieses Produktes der Summenformel C₂₆H₃₃N₂O₂F₃ ergab:

| | | | |
|---|---|---|---|
| berechnet: | C = 57,51; | H = 7,19; | N = 6,06 % |
| gefunden: | C = 67,42; | H = 7,39: | N = 6,03 %. |

### Beispiel 5

### Herstellung von 4-Trifluoracetyl-4'-lauroyloxy-azobenzol

Die im Titel genannte Verbindung entspricht der Formel IIIa, wobei der Rest R₁ die Estergruppierung der Formel 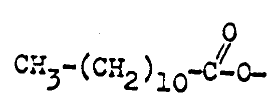
ist.

Eine Mischung aus 0,50 g (1,70 mmol) des nach dem Verfahren gemäss Beispiel 4 hergestellten 4-Trifluoracetyl-4'-hydroxy-azobenzoles und 1,00 g (4,57 mmol) an Lauroylchlorid in 5 ml Pyridin wurde bei 5° C gerührt.

Nachdem man eine Stunde lang gerührt hatte, setzte man der rot gefärbten Mischung 2 ml an Diäthyläther zu und filtrierte das gebildete Pyridinhydrochlorid ab. Dieser Niederschlag wurde mit 3 ml Diäthyläther gewaschen und die Filtrate wurden vereinigt.

Die vereinigten Filtrate wurden zunächst auf eine Säule aufgebracht, die mit 60 g 230-400 mesh SiO₂ (Fluka AG, Schweiz) gefüllt war und als Elutionsmittel wurde Aethyläther verwendet, um restliche Mengen an Pyridin zu entfernen. Dann erfolgte eine Chromatographie auf einer Säule, die mit 30 g des angeführten Siliziumdioxides gefüllt war und als Elutionsmittel wurde eine Mischung aus einem Teil Methylenchlorid und drei Teilen Hexan verwendet, um restliche Mengen der gebildeten Laurinsäure zu entfernen.

Man erhielt so 0,35 g der im Titel genannten Verbindung, die einen Schmelzpunkt von 72 - 73° C aufwies. Die Ausbeute entsprach 43 % der Theorie.

Das Produkte wurde aus Diäthyläther umkristallisiert, wobei man 0,27 g des reinen Produktes in Form von roten Plättchen erhielt, die einen Schmelzpunkt von 72,5 - 74° C aufwiesen.

Im Infrarotspektrum, aufgenommen in Chloroform, zeigte das Produkt eine Absorption bei 1715 cm⁻¹, entsprechend der Gruppierung der Formel

-COCF₃.

Das kernmagnetische Resonanzspektrum,
¹H-NMR (300, CDCl₃) ergab:
8,22-8,24 (m, 2H); 7,98-8,03 (m, 4H); 7,25-7,30 (m, 2H); 2,60 (t, J = 7,4, 2H); 1,73-1,83 (m, 2H); 1,25-1,50 (m, 16H); 0,89 (t, J = 6,7, 3H).

Das Massenspektrum ergab M⁺, m/e = 476.

Die Elementaranalyse dieses Produktes der Summenformel C₂₆H₃₁N₂O₃F₃ ergab die folgenden Werte:

| | | | |
|---|---|---|---|
| berechnet: | C = 65,53; | H = 6,56; | N = 5,88 % |
| gefunden: | C = 65,34; | H = 6,63; | N = 5,95 %. |

### Beispiel 6

### Herstellung von 4-Trifluoracetyl-4'-bis (2-butyryloxyäthyl)amino-azobenzol

Die im Titel genannte Verbindung entspricht der Formel IIIa wobei der Rest R₁ eine substituierte Dialkylaminogruppe der Formel 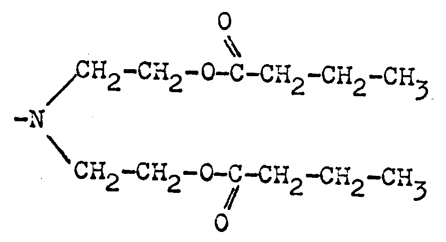
ist.

Zu einer Lösung von 0,50 g (2,64 mmol) an p-Trifluoracetyl-anilin in 4 ml Essigsäure setzte man 0,18 g (2,61 mmol) an Natriumnitrit zu. Es wurde eine geringe Menge an Stickstoffgas gebildet und das Diazoniumsalz fiel aus. Nach 10 Minuten wurde die tief gelb gefärbte Suspension weiterverwendet. Vorher war aus n-Buttersäure-chlorid und N-Phenyldiäthanolamin das N,N-Bis(2-butyryloxy-äthyl)anilin hergestellt worden. 0,93 g (2,77 mmol) an diesem N,N-Bis(2-butyryloxyäthyl)anilin wurden in 6 ml Essigsäure gelöst und die dunkelgelbe Suspension des Diazoniumsalzes wurde zugesetzt. Man liess drei Stunden stehen und verdünnte dann die rote Lösung mit 50 ml Diäthyläther. Die ätherische Lösung wurde dreimal mit 10 ml Wasser ausgeschüttelt und nach dem Abdampfen der ätherischen Lösung erhielt man ein ziegelrotes Oel, das beim Stehenlassen langsam kristallisierte.

Dieses Oel wurde auf eine Chromatographiesäule aufgebracht, die mit 60 g Siliziumdioxid (230-400 mesh SiO₂, Fluka AG, Schweiz) gefüllt war. Als Elutionsmittel wurde Diäthyläther verwendet und es wurden zwei Fraktionen aufgefangen. Die erste Fraktion enthielt 1,36 g (2,61 mmol) der im Titel genannten Verbindung und diese wurde in Form von feinen roten Nadeln isoliert, die einen Schmelzpunkt von 53 - 56° C aufwiesen. Die Ausbeute entsprach 99 % der theoretischen Ausbeute.

Die zweite Fraktion enthielt 5,1 mg von dem als Nebenprodukt gebildeten 4-Trifluoracetyl-4'-[N-(2-butyryloxyäthyl)-N-(2-hydroxy-äthyl)amino]-azobenzol als Oel. In diesem Nebenprodukt war also keine der beiden estersubstituierten Alkylgruppen des Dialkylaminosubstituenten verseift worden.

Die feinen roten Nadeln der im Titel genannten Verbindung wurden aus 20 ml Hexan umkristallisiert, wobei man 1,01 g des reinen Produktes erhielt, das einen Schmelzpunkt von 57° C aufwies.

Dieses reine Produkt zeigte im Infrarotspektrum, aufgenommen in Chloroform, eine Absorption bei 1715 cm⁻¹, entsprechend der Gruppe der Formel

-COCF₃.

Das kernmagnetische Resonanzspektrum,
¹H-NMR (300, CDCl₃) ergab:
8,18 (m, 2H); 7,91-7,96 (m, 4H); 6,89 (m, 2H); 4,32 (t, J = 6,3, 4H); 3,75 (t, J = 6,2, 4H); 2,29 (t, J = 7,4 4H); 1,58-1,70 (m, 4H); 0,94 (t, J = 7,4 4H).

Das Massenspektrum ergab M⁺ m/e 521.

Die Elementaranalyse dieses Produktes der Summenformel C₂₆H₃₀N₃O₅F₃ lieferte:

| | | | |
|---|---|---|---|
| berechnet: | C = 59,88; | H = 5,80; | N = 8,06 % |
| gefunden: | C = 59,83; | H = 5,94; | N = 7,87 %. |

### Beispiel 7

### Herstellung von 4-Trifluoracetyl-azobenzol

Die im Titel genannte Verbindung entspricht der Formel IIIa, wobei R₁ ein Wasserstoffatom ist.

Diese Verbindung wurde nach dem im Beispiel 3 beschriebenen Verfahren hergestellt, wobei jedoch das aus dem p-Trifluoracetyl-anilin hergestellte Diazoniumsalz mit Benzol gekuppelt wurde.

### Beispiel 8

### Herstellung von Addukten von Verbindungen der Formel II an Carbonatanionen

Die Verbindungen der Formel II, beispielsweise die Verbindungen der Formel III, bzw. IIIa, haben lipophile Eigenschaften und die Carbonataddukte der Verbindungen der Formel II haben ebenfalls lipophile Eigenschaften. Deshalb wurde zur Herstellung der Carbonataddukte ein ebenfalls gewisse lipophile Eigenschaften aufweisendes Carbonat verwendet.

Das eingesetzte Carbonat war das Bis(tridodecylmethylammonium)carbonat, welches in der Veröffentlichung von M.E. Meyerhoff, E. Pretsch, D.H. Welti und W. Simon in Anal. Chem. 59 (1987) Seite 144 und ff beschrieben ist. Dieses Carbonat ist ein ziemlich lipophiles farbloses Oel, das in den meisten organischen Lösungsmitteln gut löslich ist.

Die Herstellung der Addukte erfolgte, indem man die entsprechende Verbindung der Formel II mit dem lipophilen Carbonatsalz in dem entsprechenden Lösungsmittel vermischte.

Es wurden die Carbonataddukte des nach dem Verfahren gemäss Beispiel 4 hergestellten 4-Trifluoracetyl-4'-dodecyloxyazobenzoles und des nach dem Verfahren gemäss Beispiel 6 hergestellten 4-Trifluoracetyl-4'-bis(2-butyryloxyäthyl)amino-azobenzoles hergestellt. Durch die Wechselwirkung des Carbonataniones mit der Carbonylgruppe dieser Verbindungen der Formel IIIa wurde die Konjugation der Carbonylgruppe mit dem aromatischen System aufgehoben und, wie zu erwarten war, trat deshalb eine Verschiebung der Absorption des Carbonatadduktes im Vergleich zu der freien Carbonylverbindung in Richtung zu kürzeren Wellenlängen auf, also eine hypsochrome Verschiebung.

In den folgenden Tabellen I und II ist in der Spalte A das Absorptionsmaximum in nm der entsprechenden Verbindung der Formel IIIa angegeben und in Klammern direkt daneben der molare Extinktionskoeffizient.

In der Spalte B ist das Absorptionsmaximum des Carbonatadduktes in nm angeführt und in Klammern direkt daneben der molare Extinktionskoeffizient des Carbonatadduktes.

In der Spalte C ist die Verschiebung des Absorptionsmaximums in nm des Carbonatadduktes in Richtung zu kürzeren Wellenlängen, verglichen mit der entsprechenden Verbindung der Formel IIIa angeführt.

**Tabelle I**

| Verbindung gemäss Beispiel 4 | | | |
|---|---|---|---|
| Lösungsmittel | A | B | C |
| Aceton | 360(26000) | 348(30900) | -12 |
| Diäthyl-äther | 373(30900) | 352(31600) | -21 |
| Hexan | 372(32100) | 347(29400) | -25 |

**Tabelle II**

| Verbindung gemäss Beispiel 6 | | | |
|---|---|---|---|
| Lösungsmittel | A | B | C |
| Aceton | 452(27100) | 408(31100) | -44 |
| Diäthyl-äther | 445(34400) | 397(31500) | -48 |
| Hexan | 437(34700) | 395(31600) | -42 |

## Patentansprüche (Patentansprüche für folgende(n) Vertragsstaat(en): AT, BE, CH, DE, FR, GB, IT, LI, LU, NL, SE)

1. Addukte aus Anionen und Ketoverbindungen, dadurch gekennzeichnet, dass die Anionen solche von Oxasäuren sind und dass die Ketoverbindungen Monoketone der Formel I 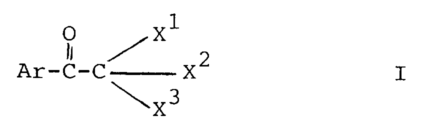 sind, in welcher
Ar ein substituierter oder unsubstituierter, ein- oder mehrkerniger, aromatischer oder ein substituierter oder unsubstituierter, ein- oder mehrkerniger, heterocyclischer Rest aromatischen Charakters ist, vorzugsweise ein Benzolkern, ein Naphthalin, ein Anthracen, ein Thiophen, ein Furan, ein Pyrrol, ein Indol, ein Benzofuran, ein Benzothiophen, ein Pyridin, ein Chinolin, ein Pyrazin oder ein Triazin ist, wobei die aromatischen oder heteroaromatischen Ringsysteme als Substituenten eine oder mehrere der folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen tragen können und wobei
X¹, X² und X³ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und unter der weiteren Voraussetzung, dass dann, wenn Ar Alkoxycarbonylphenyl oder Alkylphenyl ist, nicht sämtliche Reste X¹, X² und X³ die Bedeutung von Fluoratomen haben dürfen.

2. Addukte nach Anspruch 1, dadurch gekennzeichnet, dass die Ketoverbindungen der Formel I solche sind, in welchen die Ketogruppe ein Teil eines Chromophoren ist oder dass die Ketoverbindungen eine fluoreszierende Gruppe aufweisen, wobei sich diese Ketoverbindungen von dem Addukt derselben mit einem Anion einer Oxasäure bezüglich der Absorption im sichtbaren Bereich oder im ultraviolletten Bereich unterscheiden oder wobei durch die Adduktbildung der Ketoverbindung mit dem Anion der Oxasäure eine Fluoreszenz hervorgerufen oder eine Fluoreszenz gelöscht wird.

3. Addukte nach Anspruch 1 oder 2, dadurch gekennzeichnet, dass ihre Ketoverbindungen Azoverbindungen der Formel II 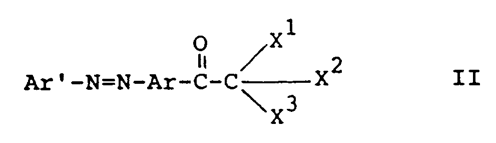 sind, in welcher die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und
X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und
wobei sich die Ketoverbindungen der Formel II von den Addukten derselben mit den Anionen der Oxasäuren bezüglich der Absorption im sichtbaren Bereich oder im Ultraviolettbereich unterscheiden.

4. Addukte nach einem der Ansprüche 1 - 3, dadurch gekennzeichnet, dass das Anion der Oxasäure, welches mit der Ketoverbindung das Addukt, vorzugsweise einen Komplex, bildet, CO₃⁻⁻, HCO₃⁻, SO₄⁻⁻, HSO₄⁻, SO₃⁻⁻, HSO₃⁻, PO₄⁻⁻⁻, HPO₄⁻⁻, H₂PO₄⁻, Cl0₃⁻, Cl0₄⁻, BrO₃⁻, JO₃⁻, NO₃⁻, NO₂⁻, CrO₄⁻⁻, HCrO₄⁻, Cr₂O₇⁻⁻, HCr₂O₇⁻ oder ein Anion einer Carbonsäure, Hydroxycarbonsäure, Ketocarbonsäure oder Aminocarbonsäure, eines Peptides, oder eines organischen Derivates der Phosphorsäure, insbesondere eines Esters der Phosphorsäure, der Diphosphorsäure oder Triphosphorsäure, beispielsweise der Adenylsäure, des Adenosindiphosphats oder Adenosintriphosphats, ist.

5. Addukte nach Anspruch 3 oder 4, dadurch gekennzeichnet, dass in der Azoverbindung der Formel II die Reste
Ar und Ar' unabhängig voneinander die Bedeutung der aromatischen Ringsysteme Benzol, Naphthalin oder Anthracen oder die Bedeutung der heterocyclischen Reste aromatischen Charakters Thiophen, Furan, Pyrrol, Indol, Benzofuran, Benzothiophen, Pyridin, Chinolin, Pyrazin oder Triazin aufweisen, wobei
die genannten aromatischen oder heteroaromatischen Ringsysteme als Substituenten eine oder mehrere oder folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen tragen können.

6. Addukte nach einem der Ansprüche 3 bis 5, dadurch gekennzeichnet, dass die Azo-ketokomponente des Adduktes die Formel III 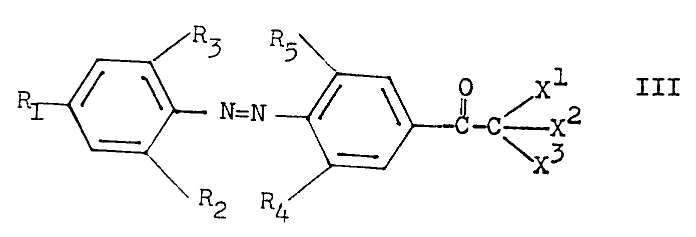 aufweist, in welcher die Substituenten
R₁, R₂, R₃, R₄ und R₅ unabhängig voneinander die Bedeutung von Wasserstoffatomen, Hydroxygruppen, Alkyläthergruppen, Aryläthergruppen, veresterten Hydroxygruppen, Aminogruppen, Alkylaminogruppen, deren Alkylrest gegebenenfalls substituiert ist, Dialkylaminogruppen, deren Alkylgruppen gegebenenfalls substituiert sind, Arylaminogruppen, deren Arylrest gegebenenfalls substituiert ist, Nitrogruppen, gegebenenfalls substituierten Alkylgruppen, gegebenenfalls substituierte Alkenylgruppen, gegebenenfalls substituierte Alkinylgruppen oder substituierte oder unsubstituierte ein- oder mehrkernige aromatischen oder substituierten oder unsubstituierten ein- oder mehrkernigen heterocyclischen Resten aromatischen Charakters aufweisen.

7. Addukte nach einem der Ansprüche 1 bis 6, dadurch gekennzeichnet, dass in der Ketokomponente der Formeln I, II, beziehungsweise III der Addukte in der Gruppe der Formel 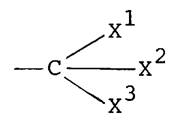 mindestens zwei der Reste X¹, X² und X³ stark elektronenanziehende Substituenten, ausgewählt aus der Gruppe Fluoratome, Chloratome, Bromatome oder Nitrogruppen sind, und wobei ferner in denjenigen Verbindungen der Formel I, in welchen Ar eine andere Bedeutung besitzt, als Alkoxycarbonylphenyl oder Alkylphenyl, und in den Verbindungen der Formeln II und III vorzugsweise sämtliche Reste X¹, X² und X³ für Fluoratome stehen.

8. Verfahren zur Herstellung eines Adduktes aus Anionen und Ketoverbindungen gemäss Anspruch 1, dadurch gekennzeichnet, dass man das Anion einer Oxasäure mit der Ketoverbindung in Berührung bringt, wobei sich das Addukt bildet.

9. Verfahren nach Anspruch 8, dadurch gekennzeichnet, dass man ein Addukt gemäss einem der Ansprüche 2 bis 7 herstellt.

10. Ketoverbindungen zur Durchführung des Verfahrens gemäss Anspruch 8, dadurch gekennzeichnet, dass sie Azoketoverbindungen der Formel II 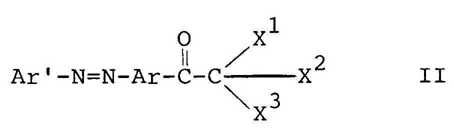 sind, in welcher die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und
X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituengen X¹, X² und X³ ein stark elektronenanziehender Substituent, ausgewählt aus der Gruppe Fluoratome, Chloratome, Bromatome oder Nitrogruppen ist, und wobei in den Azoketoverbindungen der Formel II vorzugsweise mindestens zwei der Substituenten X¹, X² und X³ derartig stark elektronenanziehende Substituenten sind, und speziell bevorzugt sämtliche der Substituenten X¹, X² und X³ Fluoratome bedeuten.

11. Azoketoverbindungen gemäss Anspruch 10, dadurch gekennzeichnet, dass in den Verbindungen der Formel II die Reste
Ar und Ar' unabhängig voneinander die aromatischen Ringsysteme Benzol, Naphthalin oder Anthracen oder die heterocyclischen Reste aromatischen Charakters Thiophen, Furan, Pyrrol, Indol, Benzofuran, Benzothiophen, Pyridin, Chinolin, Pyrazin oder Triazin sind, welche
ein oder mehrere Substituenten aus der folgenden Gruppe: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen, tragen können.

12. Azoketoverbindungen gemäss Anspruch 11, gekennzeichnet durch die Formel III 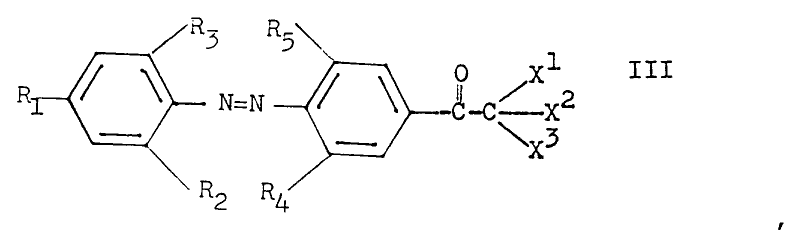 in welcher die Substituenten
R₁, R₂, R₃, R₄ und R₅ unabhängig voneinander die Bedeutung von Wasserstoffatomen, Hydroxygruppen, Alkyläthergruppen, Aryläthergruppen, veresterten Hydroxygruppen, Aminogruppen, Alkylaminogruppen, deren Alkylrest gegebenenfalls substituiert ist, Dialkylaminogruppen, deren Alkylgruppen gegebenenfalls substituiert sind, Arylaminogruppen, deren Arylrest gegebenenfalls substituiert ist, Nitrogruppen, gegebenenfalls substituierte Alkylgruppen, gegebenenfalls substituierte Alkenylgruppen, gegebenenfalls substituierte Alkinylgruppen oder substituierte oder unsubstituierte ein- oder mehrkernige aromatische oder substituierte oder unsubstituierte ein- oder mehrkernige heterocyclische Reste aromatischen Charakters aufweisen.

13. Azoketoverbindungen gemäss Anspruch 12, dadurch gekennzeichnet, dass in ihnen die Reste
R₄ und R₅ Wasserstoffatome sind und
ein oder zwei der Reste R₁, R₂ und R₃ die Bedeutung einer Hydroxygruppe, einer veresterten Hydroxygruppe, einer Nitrogruppe, einer Aminogruppe, einer Monoalkylaminogruppe mit gegebenenfalls substituierter Alkylgruppe, einer Dialkylaminogruppe mit gegebenenfalls substituierter Alkylgruppe oder einer Arylaminogruppe, beziehungsweise Diarylaminogruppe aufweisen, wobei der, beziehungsweise die Arylreste gegebenenfalls Substituenten tragen, und wobei vorzugsweise der Rest R₁ einer der erwähnten Substituenten ist, während die Reste R₂ und R₃ die Bedeutung von Wasserstoffatomen aufweisen.

14. Verfahren zur Herstellung der neuen Azoketoverbindungen gemäss Anspruch 10, dadurch gekennzeichnet, dass man die Azoketoverbindungen der Formel II 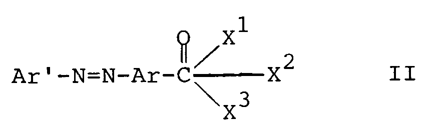 herstellt, in welcher die Reste
Ar, Ar', X¹, X² und X³ die im Anspruch 10 angegebene Bedeutung besitzen, indem man entweder
ein primäres aromatisches oder heteroaromatisches Amin der Formel V 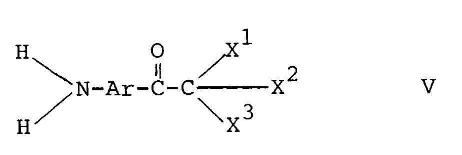 diazotiert und einer Azokupplung mit einer aromatischen oder heteroaromatischen Verbindung der Formel VI
Ar'-H VI
unterwirft, wobei sich die Azoverbindung der Formel II bildet,
oder dass man ein primäres aromatisches oder heteroaromatisches Amin der Formel VII 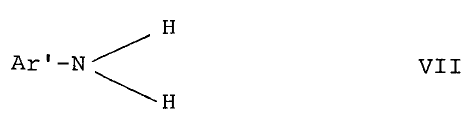 diazotiert und dieses einer Azokupplung mit einer aromatischen oder heteroaromatischen Verbindung der Formel VIII 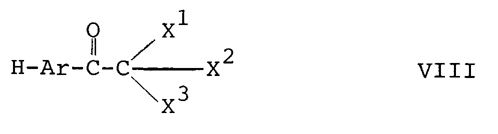 unterwirft, wobei sich die Azoverbindung der Formel II bildet.

15. Verfahren nach Anspruch 14, dadurch gekennzeichnet, dass man die in den Ansprüchen 11 bis 13 definierten Azo-Ketoverbindungen herstellt.

16. Verwendung der Ketoverbindungen der im Anspruch 1 angegebenen Formel I zum Nachweis von Anionen von Oxasäuren, indem man die Ketoverbindungen der Formel I mit den nachzuweisenden Anionen in Berührung bringt, unter Bildung der im Anspruch 1 definierten Addukte.

17. Verwendung nach Anspruch 16, dadurch gekennzeichnet, dass man als Anionen von Oxasäuren CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, JO₃⁻, NO₃⁻, NO₂⁻, CrO₄⁻⁻, HCrO₄⁻, Cr₂O₇⁻⁻, HCr₂O₇⁻ oder Anionen von Carbonsäuren, Hydroxycarbonsäuren, Ketocarbonsäuren, Aminocarbonsäuren, Peptiden, oder organischen Derivaten der Phosphorsäure, insbesondere Estern der Phosphorsäure, der Diphosphorsäure oder Triphosphorsäure, beispielsweise Adenylsäure, Adenosindiphosphat oder Adenosintriphosphat, verwendet.

18. Verwendung nach Anspruch 16 oder 17, dadurch gekennzeichnet, dass man die Ketoverbindung als ionenselektive Komponente eines ionenselektiven Teiles zur Bestimmung der Konzentration der Anionen der Oxasäuren einsetzt, beispielsweise als ionenselektive Komponente einer Membran einer ionenselektiven Elektrode zur Bestimmung der Konzentration der Anionen, wobei der ionenselektive Teil gegebenenfalls als weitere Komponente eine quaternäre Ammoniumverbindung mit mindestens einer lipophilen aliphatischen Kohlenwasserstoffkette mit mindestens acht Kohlenstoffatomen enthält.

19. Verwendung nach einem der Ansprüche 16 bis 18, dadurch gekennzeichnet, dass man eine Azo-ketoverbindung der Formel II gemäss der in einem der Ansprüche 3 oder 5 - 7 gegebenen Definition verwendet.

20. Verwendung nach Anspruch 16 oder 17, dadurch gekennzeichnet, dass man eine Ketoverbindung gemäss der in Anspruch 2 oder 3 gegebenen Definition verwendet, in welcher die Ketogruppe ein Teil eines Chromophoren ist oder die Ketoverbindung eine fluoreszierende Gruppe aufweist, und wobei diese Ketoverbindungen als Farbindikatoren verwendet werden, bei denen durch die Reaktion des Anions der Oxasäuren mit der Ketoverbindung eine Verschiebung der Absorption der Ketoverbindung im sichtbaren Bereich oder im Ultraviolettbereich auftritt oder eine Fluoreszenz hervorgerufen oder eine Fluoreszenz gelöscht wird.

21. Verwendung nach Anspruch 16, dadurch gekennzeichnet, dass man die Ketoverbindung als Komponente einer Testvorrichtung verwendet, wobei die Ketoverbindung sich auf einem, bzw. in einem Trägermaterial befindet, beispielsweise einem Kunststoffträgermaterial oder einem Trägermaterial aus Papier, oder wobei die Testvorrichtung ein Sensor ist, der die Ketoverbindung enthält und der bei Berührung mit Anionen von Oxasäuren eine Farbveränderung im sichtbaren Bereich oder im ultravioletten Bereich oder ein Auftreten einer Fluoreszenz oder ein Löschen der Fluoreszenz zeigt, und wobei die Sensoren vorzugsweise Sensoren von Optoden sind, bei denen der Sensor in schwer zugängliche Bereiche eingeführt werden kann und die Änderung der Farbe oder Fluoreszenz bei Kontakt mit den Anionen der Oxasäuren über einen Transmitter, beispielsweise einen Lichtleiter, beobachtet wird.

22. Verwendung nach Anspruch 20 oder 21, dadurch gekennzeichnet, dass man eine Ketoverbindung gemäss der in einem der Ansprüche 2 oder 3 oder 5 bis 7 gegebenen Definition verwendet.

## Patentansprüche (Patentansprüche für folgende(n) Vertragsstaat(en): ES)

1. Verfahren zur Herstellung von Addukten, Chelaten, beziehungsweise Komplexen aus Anionen und Ketoverbindungen, dadurch gekennzeichnet, dass man Anionen von Oxasäuren mit Ketoverbindungen in Berührung bringt, wobei diese Ketoverbindungen Monoketone der Formel I 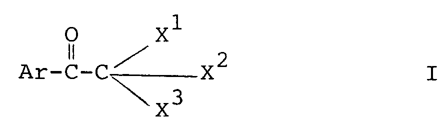 sind, in welcher
Ar ein substituierter oder unsubstituierter, ein- oder mehrkerniger, aromatischer oder ein substituierter oder unsubstituierter, ein- oder mehrkerniger, heterocyclischer Rest aromatischen Charakters ist, vorzugsweise ein Benzolkern, ein Naphthalin, ein Anthracen, ein Thiophen, ein Furan, ein Pyrrol, ein Indol, ein Benzofuran, ein Benzothiophen, ein Pyridin, ein Chinolin, ein Pyrazin oder ein Triazin ist, wobei die aromatischen oder heteroaromatischen Ringsysteme als Substituenten eine oder mehrere der folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen tragen können und wobei
X¹, X² und X³ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und unter der weiteren Voraussetzung, dass dann, wenn Ar Alkoxycarbonylphenyl oder Alkylphenyl ist, nicht sämtliche Reste X¹,X² und X³ die Bedeutung von Fluoratomen haben dürfen.

2. Verfahren nach Anspruch 1, dadurch gekennzeichnet, dass man Addukte mit solchen Ketoverbindungen der Formel I herstellt, in welcher die Ketoverbindungen eine fluoreszierende Gruppe aufweisen, wobei sich diese Ketoverbindungen von dem Addukt derselben mit einem Anion einer Oxasäure bezüglich der Absorption im sichtbaren Bereich oder im ultravioletten Bereich unterscheidet oder wobei durch die Adduktbildung der Ketoverbindung mit dem Anion der Oxasäure eine Fluoreszenz hervorgerufen oder eine Fluoreszenz gelöscht wird.

3. Verfahren nach Anspruch 1 oder 2, dadurch gekennzeichnet, dass man zur Herstellung der Addukte Ketoverbindungen verwendet, die Azoverbindungen der Formel II 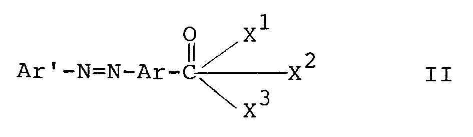 sind, in welcher die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und
X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und
wobei sich die Ketoverbindungen der Formel II von den Addukten derselben mit den Anionen der Oxasäuren bezüglich der Absorption im sichtbaren Bereich oder im Ultraviolettbereich unterscheiden.

4. Verfahren nach einem der Ansprüche 1 bis 3, dadurch gekennzeichnet, dass man als Anion der Oxasäure, welches mit der Ketoverbindung das Addukt, vorzugsweise einen Komplex, bildet, CO₃⁻⁻, HCO₃⁻, SO₄⁻⁻, HSO₄⁻, SO₃⁻⁻, HSO₃⁻, PO₄⁻⁻⁻, HPO₄⁻⁻, H₂PO₄⁻, Cl0₃⁻, Cl0₄⁻, BrO₃⁻, JO₃⁻, NO₃⁻, NO₂⁻, CrO₄⁻⁻, HCrO₄⁻, Cr₂O₇⁻⁻, HCr₂O7⁻ oder ein Anion einer Carbonsäure, Hydroxycarbonsäure, Ketocarbonsäure oder Aminocarbonsäure, eines Peptides, oder eines organischen Derivates der Phosphorsäure, insbesondere eines Esters der Phosphorsäure, der Diphosphorsäure oder Triphosphorsäure, beispielsweise der Adenylsäure, des Adenosindiphosphats oder Adenosintriphosphats, verwendet.

5. Verfahren nach Anspruch 3 oder 4, dadurch gekennzeichnet, dass man eine Azoverbindung der Formel II verwendet, in welcher die Reste
Ar und Ar' unabhängig voneinander die Bedeutung der aromatischen Ringsysteme Benzol, Naphthalin oder Anthracen oder die Bedeutung der heterocyclischen Reste aromatischen Charakters Thiophen, Furan, Pyrrol, Indol, Benzofuran, Benzothiophen, Pyridin, Chinolin, Pyrazin oder Triazin aufweisen, wobei
die genannten aromatischen oder heteroaromatsichen Ringsysteme als Substituenten eine oder mehrere der folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen tragen können.

6. Verfahren nach einem der Ansprüche 3 bis 5, dadurch gekennzeichnet, dass man ein Addukt mit einer Azo-ketoverbindung der Formel III 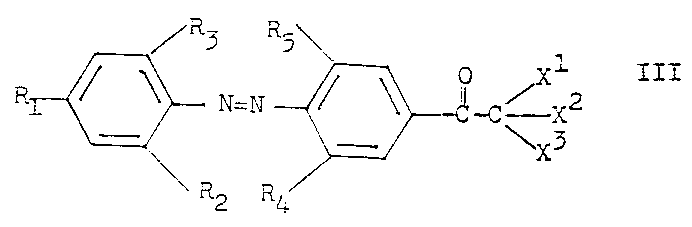 herstellt, in welcher die Substituenten
R₁, R₂, R₃, R₄ und R₅ unabhängig voneinander die Bedeutung von Wasserstoffatomen, Hydroxygruppen, Alkyläthergruppen, Aryläthergruppen, veresterten Hydroxygruppen, Aminogruppen, Alkylaminogruppen, deren Alkylrest gegebenenfalls substituiert ist, Dialkylaminogruppen, deren Alkylgruppen gegebenenfalls substituiert sind, Arylaminogruppen, deren Arylrest gegebenenfalls substituiert ist, Nitrogruppen, gegebenenfalls substituierten Alkylgruppen, gegebenenfalls substituierte Alkenylgruppen, gegebenenfalls substituierte Alkinylgruppen oder substituierte oder unsubstituierte einoder mehrkernige aromatischen oder substituierten oder unsubstituierten ein- oder mehrkernigen heterocyclischen Resten aromatischen Charakters ausweisen.

7. Verfahren nach einem der Ansprüche 1 bis 6, dadurch gekennzeichnet, dass man zur Herstellung der Addukte eine Ketokomponente der Formeln I, II, beziehungsweise III verwendet, in welcher in der Gruppe der Formel 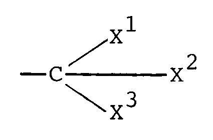 mindestens zwei der Reste X¹, X² und X³ stark elektronenanziehende Substituenten, ausgewählt aus der Gruppe Fluoratome, Chloratome, Bromatome oder Nitrogruppen, sind, und wobei ferner in denjenigen Verbindungen der Formel I, in welchen Ar eine andere Bedeutung besitzt, als Alkoxycarbonylphenyl oder Alkylphenyl, und in den Verbindungen der Formeln II und III vorzugsweise sämtliche Reste X¹, X² und X³ für Fluoratome stehen.

8. Verfahren zur Herstellung von Ketoverbindungen der Formel I 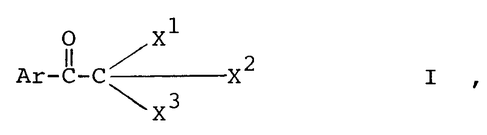 in welcher
Ar ein substituierter oder unsubstituierter, ein- oder mehrkerniger, aromatischer oder ein substituierter oder unsubstituierter, ein- oder mehrkerniger, heterocyclischer Rest aromatischen Charakters ist, vorzugsweise ein Benzolkern, ein Naphthalin, ein Anthracen, ein Thiophen, ein Furan, ein Pyrrol, ein Indol, ein Benzofuran, ein Benzothiophen, ein Pyridin, ein Chinolin, ein Pyrazin oder ein Triazin ist, wobei die aromatischen oder heteroaromatischen Ringsysteme als Substituenten eine oder mehrere der folgenden Gruppen: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substituierte oder unsubstituierte Alkenylgruppen, substituierte oder unsubstituierte Alkinylgruppen, substituierte oder unsubstituierte Cycloalkylgruppen und substituierte oder unsubstituierte Arylgruppen tragen können und wobei
X¹, X² und X³ unabhängig voneinander Wasserstoffatome, Alkylgruppen, Alkenylgruppen, Alkinylgruppen, Fluoratome, Chloratome, Bromatome oder Nitrogruppen bedeuten, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist, und unter der weiteren Voraussetzung, dass dann, wenn Ar Alkoxycarbonylphenyl oder Alkylphenyl ist, nicht sämtliche Reste X¹, X² und X³ die Bedeutung von Fluoratomen haben dürfen,
dadurch gekennzeichnet, dass man eine aromatische, beziehungsweise heteroaromatische Verbindung der Formel IX
ArH IX
mit einem Acylhalogenid der Formel X 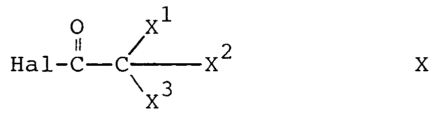 nach dem Verfahren der Friedel-Crafts-Acylierung umsetzt und die gebildeten Ketoverbindungen der Formel I isoliert.

9. Verfahren nach Anspruch 8, dadurch gekennzeichnet, dass man Azoketoverbindungen der Formel II 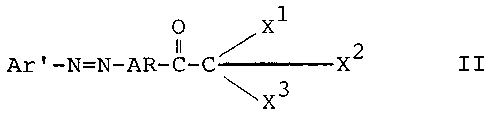 herstellt, in welcher die Reste
Ar und Ar' unabhängig voneinander substituierte oder unsubstituierte, ein- oder mehrkernige, aromatische oder substituierte oder unsubstituierte, ein- oder mehrkernige heterocyclische Reste aromatischen Charakters bedeuten und X¹, X² und X³ unabhängig voneinander Wasserstoffatome, Alkylgruppen, Alkenylgruppen, Alkinylgruppen, Fluoratome, Chloratome, Bromatome oder Nitrogruppen sind, unter der Voraussetzung, dass jedoch mindestens einer der Substituenten X¹, X² und X³ ein stark elektronenanziehender Substituent, ausgewählt aus der Gruppe Fluoratome, Chloratome, Bromatome oder Nitrogruppen ist,
indem man
entweder ein primäres aromatisches oder heteroaromatisches Amin der Formel V 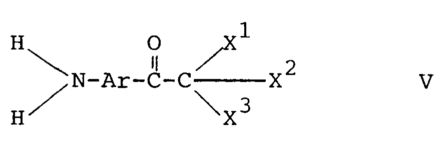 diazotiert und einer Azokupplung mit einer aromatischen oder heteroaromatischen Verbindung der Formel VI
Ar'-H VI
unterwirft, wobei sich die Azoverbindung der Formel II bildet,
oder indem man ein primäres aromatisches oder heteroaromatisches Amin der Formel VII 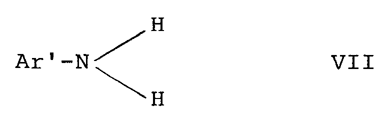 diazotiert und dieses einer Azokupplung mit einer aromatischen oder heteroaromatischen Verbindung der Formel VIII 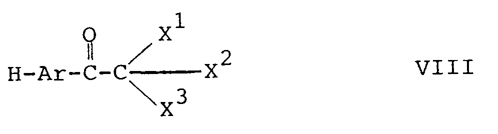 unterwirft, wobei sich die Azoverbindung der Formel II bildet.

10. Verfahren nach Anspruch 9, dadurch gekennzeichnet, dass man Azoketoverbindungen der Formel II herstellt, in welchen die Reste
Ar und Ar' unabhängig voneinander die aromatischen Ringsysteme Benzol, Naphthalin oder Anthracen oder
die heterocyclischen Reste aromatischen Charakters Thiophen, Furan, Pyrrol, Indol, Benzofuran, Benzothiophen, Pyridin, Chinolin, Pyrazin oder Triazin sind, welche
ein oder mehrere Substituenten aus der folgenden Gruppe: Halogenatome, Nitrogruppen, Alkylgruppen, Azogruppen, halogensubstituierte Alkylgruppen, Aethergruppierungen, Carbonsäureestergruppierungen, Hydroxygruppen, veresterte Hydroxygruppen, Aminogruppen, Alkylaminogruppen, in welchen der Alkylrest gegebenenfalls Substituenten trägt, Dialkylaminogruppen, in welchen die Alkylreste gegebenenfalls Substituenten tragen, Arylaminogruppen, beziehungsweise Diarylaminogruppen, in welchen die Arylreste gegebenenfalls Substituenten tragen, Cycloalkylaminogruppen, beziehungsweise Dicycloalkylaminogruppen, in denen die Cycloalkylgruppen gegebenenfalls Substituenten tragen, substituierte Alkylgruppen, substitutierte oder unsubstituierte Alkenlylgruppen, substituierte oder unsubstituierte Alkinylgruppen, substituierte oder unsubstituierte Cycloalkylgruppen und substituierte oder unsubstituierte Arylgruppen, tragen können.

11. Verfahren nach Anspruch 10, dadurch gekennzeichnet, dass man Azoketoverbindungen der Formel III 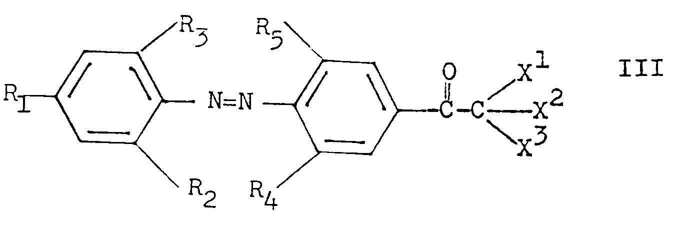 herstellt, in welchen die Substituenten
R₁, R₂, R₃, R₄ und R₅ unabhängig voneinander die Bedeutung von Wasserstoffatomen, Hydroxygruppen, Alkyläthergruppen, Aryläthergruppen, veresterten Hydroxygruppen, Aminogruppen, Alkylaminogruppen, deren Alkylrest gegebenenfalls substituiert ist, Dialkylaminogruppen, deren Alkylgruppen gegebenenfalls substituiert sind, Arylaminogruppen, deren Arylrest gegebenenfalls substituiert ist, Nitrogruppen, gegebenenfalls substituierte Alkylgruppen, gegebenenfalls substituierte Alkenylgruppen, gegebenenfalls substituierte Alkinylgruppen oder substituierte oder unsubstituierte einoder mehrkernige aromatische oder substituierte oder unsubstituierte ein- oder mehrkernige heterocyclische Reste aromatischen Charakters aufweisen.

12. Verfahren nach Anspruch 11, dadurch gekennzeichnet, dass man Azoketoverbindungen der Formel III herstellt, in welchen die Reste
R₄ und R₅ Wasserstoffatome sind und
ein oder zwei der Reste R₁, R₂ und R₃ die Bedeutung einer Hydroxygruppe, einer veresterten Hydroxygruppe, einer Nitrogruppe, einer Aminogruppe, einer Monoalkylaminogruppe mit gegebenenfalls substituierter Alkylgruppe, einer Dialkylaminogruppe mit gegebenenfalls substituierter Alkylgruppe oder einer Arylaminogruppe, beziehungsweise Diarylaminogruppe aufweisen, wobei der, beziehungsweise die Arylreste gegebenenfalls Substituenten tragen, und wobei vorzugsweise der Rest R₁ einer der erwähnten Substituenten ist, während die Reste R₂ und R₃ die Bedeutung von Wasserstoffatomen aufweisen.

13. Verwendung von Ketoverbindungen der Formel I 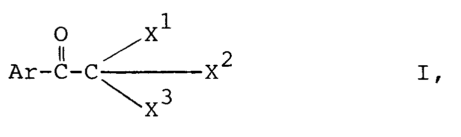 in welcher
Ar, X¹, X² und X³ die gleiche Bedeutung aufweisen, wie in Anspruch 1, zum Nachweis von Anionen von Oxasäuren, indem man die Ketoverbindungen der Formel I mit den nachzuweisenden Anionen in Berührung bringt, unter Bildung der im Anspruch 1 definierten Addukte.

14. Verwendung nach Anspruch 13, dadurch gekennzeichnet, dass man als Anionen von Oxasäuren CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, JO₃⁻, NO₃⁻, NO₂⁻, CrO₄⁻⁻, HCrO₄⁻, Cr₂O₇⁻⁻, HCr₂O₇⁻ oder Anionen von Carbonsäuren, Hydroxycarbonsäuren, Ketocarbonsäuren, Aminocarbonsäuren, Peptiden, oder organischen Derivaten der Phosphorsäure, insbesondere Estern der Phosphorsäure, der Diphosphorsäure oder Triphosphorsäure, beispielsweise Adenylsäure, Adenosindiphosphat oder Adenosintriphosphat, verwendet.

15. Verwendung nach Anspruch 13 oder 14, dadurch gekennzeichnet, dass man die Ketoverbindung als ionenselektive Komponente eines ionenselektiven Teiles zur Bestimmung der Konzentration der Anionen der Oxasäuren einsetzt, beispielsweise als ionenselektive Komponente einer Membran einer ionenselektiven Elektrode zur Bestimmung der Konzentration der Anionen, wobei der ionenselektive Teil gegebenenfalls als weitere Komponente eine quaternäre Ammoniumverbindung mit mindestens einer lipophilen aliphatischen Kohlenwasserstoffkette mit mindestens acht Kohlenstoffatomen enthält.

16. Verwendung nach einem der Ansprüche 13 bis 15, dadurch gekennzeichnet, dass man eine Azoketoverbindung der Formel II gemäss der in einem der Ansprüche 3 oder 5 - 7 gegebenen Definition verwendet.

17. Verwendung nach Anspruch 13 oder 14, dadurch gekennzeichnet, dass man eine Ketoverbindung gemäss der in Anspruch 2 oder 3 gegebenen Definition verwendet, in welcher die Ketogruppe ein Teil eines Chromophoren ist, oder die Ketoverbindung eine fluoreszierende Gruppe aufweist, und wobei diese Ketoverbindungen als Farbindikatoren verwendet werden, bei denen durch die Reaktion des Anions der Oxasäuren mit der Ketoverbindung eine Verschiebung der Absorption der Ketoverbindung im sichtbaren Bereich oder im Ultraviolettbereich auftritt oder eine Fluoreszenz hervorgerufen oder eine Fluoreszenz gelöscht wird.

18. Verwendung nach Anspruch 13, dadurch gekennzeichnet, dass man die Ketoverbindung als Komponente einer Testvorrichtung verwendet, wobei die Ketoverbindung sich auf einem, bzw. in einem Trägermaterial befindet, beispielsweise einem Kunststoffträgermaterial oder einem Trägermaterial aus Papier, oder wobei die Testvorrichtung ein Sensor ist, der die Ketoverbindung enthält und der bei Berührung mit Anionen von Oxasäuren eine Farbveränderung im sichtbaren Bereich oder im ultravioletten Bereich oder ein Auftreten einer Fluoreszenz oder ein Löschen der Fluoreszenz zeigt, und wobei die Sensoren vorzugsweise Sensoren von Optoden sind, bei denen der Sensor in schwer zugängliche Bereiche eingeführt werden kann und die Änderung der Farbe oder Fluoreszenz bei Kontakt mit den Anionen der Oxasäuren über einen Transmitter, beispielsweise einen Lichtleiter, beobachtet wird.

19. Verwendung nach Anspruch 17 oder 18, dadurch gekennzeichnet, dass man eine Ketoverbindung gemäss der in einem der Ansprüche 2 oder 3 oder 5 - 7 gegebenen Definition verwendet.

## Claims (Claims for the following Contracting State(s): AT, BE, CH, DE, FR, GB, IT, LI, LU, NL, SE)

1. Adducts from anions and keto compounds which are characterised in that the anions are of oxy acids and that the keto compounds are monoketones of Formula I 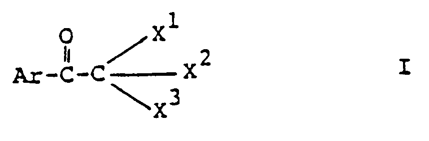 in which Ar is a substituted or unsubstituted mono- or polynuclear aromatic group or is a substituted or unsubstituted mono- or poly nuclear heterocyclic residue of an aromatic nature, preferably a benzene nucleus, naphthalene, anthracene, thiophene, furan, pyrrole, indole, benzofuran, benzothiophene, pyridine, quinoline, pyrazine or triazene, where the aromatic or heteroaromatic ring system may bear one or more of the following groups as substituents: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkyl-amino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups, in which x¹, x², x³ may represent, independently of one another, hydrogen atoms, alkyl groups, alkenyl groups, alkinyl groups, fluorine atoms, chlorine atoms, bromine atoms or nitro groups, with the condition that at least one of the substituents x¹, x², x³ is a strongly electron-attracting substituent chosen from the selection: fluorine, chlorine or bromine atoms or nitro groups and with the further condition that when Ar is an alkoxycarbonylphenyl or alkylphenyl, the groups x¹, x², x³ may not all represent fluorine atoms.

2. Adducts according to Claim 1, characterised in that the keto compounds of Formula 1 are those in which the keto group is part of a chromophore or the keto compounds contain a fluorescing group, said keto compounds differing from their adducts with an anion of an oxy acid with regard to absorption in the visible spectrum or in the ultraviolet spectrum, and fluorescence being produced or extinguished by the adduct formation of the keto compound with the anion of the oxy acid.

3. Adducts according to Claims 1 or 2, characterised in that their keto compounds are azo compounds of Formula II 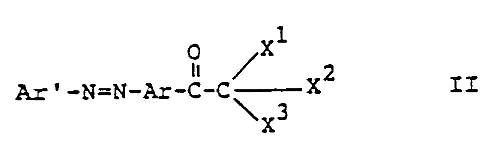 in which the radicals Ar and Ar' independently of each other represent substituted or unsubstituted mono- or polynuclear aromatic or substituted or unsubstituted mono-or polynuclear heterocyclic radicals of aromatic character and
x¹, x² and x³ independently of each other represent hydrogen atoms, alkyl groups, alkenyl groups, alkinyl groups, fluorine atoms, chlorine atoms, bromine atoms or nitro groups, with the condition that at least one of the substituents x¹, x² and x³ represents a strongly electron-attracting substituent chosen from the selection: fluorine, chlorine or bromine atoms or nitro groups, and in which the keto compounds of Formula II are distinguished from their adducts with oxy acid anions by their absorption in the visible or ultraviolet spectra.

4. Adducts according one of to Claims 1-3, characterised in that the anion of the oxy acid which forms the adduct, preferably a complex, with the keto compound is: CO₃⁻⁻, HCO₃⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, IO₃⁻, NO₃⁻, NO₂⁻, CrO₄⁻⁻, HCrO₄⁻, Cr₂O₇⁻⁻, HCr₂O₇⁻ or an anion of a carboxylic acid, hydroxycarboxylic acid, ketocarboxylic acid or of an aminocarboxylic acid, a peptide or of an organic derivative of phosphoric acid, particularly of an ester of phosphoric, diphosphoric or triphosphoric acid, for example of adenylic acid, adenosine diphosphate or adenosine triphosphate.

5. Adducts according to Claims 3 or 4, characterised in that in the azocompound of Formula II the radicals Ar and Ar' independently of each other represent the aromatic ring systems: benzene, naphthalene or anthracene, or represent the heterocyclic radicals of aromatic character: thiophene, furan, pyrrole, indole, benzofuran, benzothiophene, pyridine, quinoline, pyrazine or triazine, and the aromatic or heteroaromatic ring systems named may carry as substituents one or more of the following groups: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl radical may optionally bear substituents, dialkylamino groups in which the alkyl radicals may optionally bear substituents, arylamino groups or diarylamino groups in which the aryl radicals may optionally bear substituents, cycloalkylamino groups or dicycloalkylamino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups.

6. Adducts according to one of Claims 3 to 5, characterised in that the azoketo component of the adduct is illustrated by Formula III 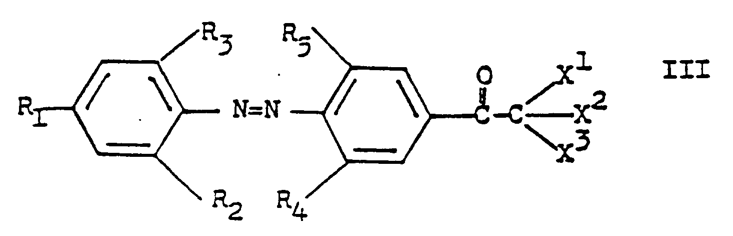 in which the substituents R₁, R₂, R₃, R₄ and R₅ independently of each other represent hydrogen atoms, hydroxy groups, alkyl ether groups, aryl ether groups, esterified hydroxy groups, amino groups, alkylamino groups in which the alkyl radical may optionally be substituted, dialkylamino groups in which the alkyl groups may optionally be substituted, arylamino groups in which the aryl radical may optionally be substituted, nitro groups, optionally substituted alkyl groups, optionally substituted alkenyl groups, optionally substituted alkinyl groups or substituted or unsubstituted mono- or polynuclear aromatic or substituted or unsubstituted mono- or polynuclear heterocyclic radicals of aromatic character.

7. Adducts according to one of Claims 1 to 6, characterised in that the adduct keto components of Formulae I, II or III possess in the grouping: 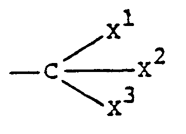 at least two of the radicals x¹, x² and x³ which are strongly electron-attracting substituents chosen from the selection fluorine, chlorine or bromine atoms or nitro groups, and furthermore in those compounds of Formula I in which Ar represents a grouping other than alkoxycarbonylphenyl or alkylphenyl, and in the compounds of Formulae II and III, preferably all of the radicals x¹, x² and x³ are fluorine atoms.

8. Process for the preparation of an adduct from anions and keto compounds according to Claim 1, characterised in that the anion of an oxy acid is brought into contact with the keto compound, thus forming the adduct.

9. Process according to Claim 8, characterised in that an adduct according to one of the Claims 2 to 7 is produced.

10. Keto compounds for carrying out the Process according to Claim 8, characterised in that they are azoketo compounds of Formula II 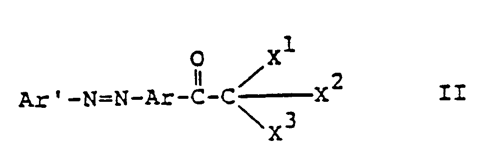 in which the radicals Ar and Ar' independently of each other represent substituted or unsubstituted mono- or polynuclear aromatic or substituted or unsubstituted mono- or polynuclear heterocyclic groups of aromatic character and x¹, x² and x³ represent independently of each other hydrogen atoms, alkyl groups, alkenyl groups, alkinyl groups, fluorine atoms, chlorine atoms, bromine atoms or nitro groups, with the provision that at least one of the substituents x¹, x² and x³ is a strongly electron-attracting substituent selected from the group fluorine, chlorine or bromine atoms or nitro groups and in which the azoketo compounds of Formula II preferably possess at least two of the substituents x¹, x² and x³ as representing strongly electron- attracting substituents, and particularly preferably where all the substituents x¹, x² and x³ represent fluorine atoms.

11. Azoketo compounds according to Claim 10, characterised in that in the compounds of Formula II the radicals Ar and Ar' denote independently of each other the aromatic rings or ring systems: benzene, naphthalene or anthracene or the heterocyclic radicals of aromatic character: thiophene, furan, pyrrole, indole, benzofuran, benzothiophene, pyridine, quinoline, pyrazine or triazine, which may bear one or more of the following groups as substitutents: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkylamino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups.

12. Azoketo compounds according to Claim 11, characterised by Formula III 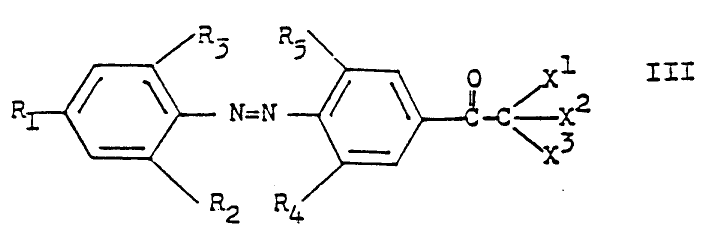 in which the substituents R₁, R₂, R₃, R₄ and R₅ independently of each other represent hydrogen atoms, hydroxy groups, alkyl ether groups, aryl ether groups, esterified hydroxy groups, amino groups, alkylamino groups in which the alkyl radical may optionally be substituted, dialkylamino groups in which the alkyl groups may optionally be substituted, arylamino groups in which the aryl radical may optionally be substituted, nitro groups, optionally substituted alkyl groups, optionally substituted alkenyl groups, optionally substituted alkinyl groups or substituted or unsubstituted mono- or polynuclear aromatic or substituted or unsubstituted mono- or polynuclear heterocyclic radicals of aromatic character.

13. Azoketo compounds according to Claim 12, characterised in that the radicals R₄ and R₅ are hydrogen atoms and one or two of the radicals R₁, R₂ and R₃ represent a hydroxy group, an esterified hydroxy group, a nitro group, an amino group, a monoalkylamino group with an optionally substituted alkyl group, a dialkylamino group with an optionally substituted alkyl group or an aryl- or diarylamino group with an optionally substituted aryl radical or radicals and where the radical R₁ is preferably one of the substituents mentioned and the radicals R₂ and R₃ represent hydrogen atoms.

14. Process for the preparation of the new azoketo compounds according to Claim 10, characterised in that the azoketo compounds of Formula II 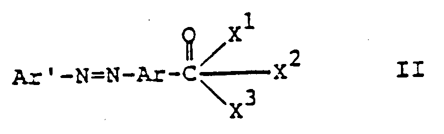 are prepared in which the radicals Ar, Ar', x¹, x² and x³ have the same significance as in Claim 10, in which a primary aromatic or heteroaromatic amine of Formula V 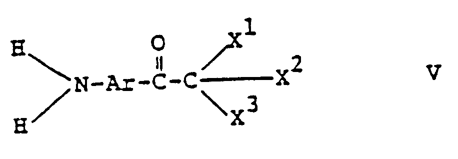 is diazotised and subjected to an azo coupling with an aromatic or heteroaromatic compound of Formula VI
Ar'-H VI
whereby the azo compound of Formula II is formed, or in that a primary aromatic or heteroaromatic amine of Formula VII 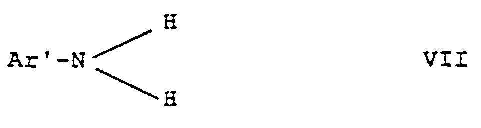 is diazotized and subjected to an azo coupling with an aromatic or heteroaromatic compound of Formula VIII 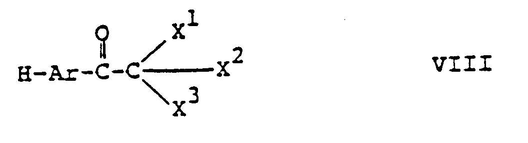 whereby the azo compound of Formula II is formed.

15. Process according to Claim 14, characterised in that the azoketo compounds defined in Claims 11 to 13 are prepared.

16. Use of the keto compounds of Formula I quoted in Claim 1 to detect the anions of oxy acids, in that the keto compounds of Formula I are brought into contact with the anions to be detected, with formation of the adducts defined in Claim 1.

17. Use according to Claim 16, characterised in that the anions of oxy acids used are CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, IO₃⁻, NO₃⁻, NO₂⁻, CrO₄⁻⁻, HCrO₄⁻, Cr₂O₇⁻⁻, HCr₂O₇⁻ or the anions of carboxylic, hydroxycarboxylic, ketocarboxylic, aminocarboxylic acids or the anions of peptides, or the anions of organic derivatives of phosphoric acid, particularly esters of phosphoric, diphosphoric or triphosphoric acids, for example adenylic acid, adenosine diphosphate or adenosine triphosphate.

18. Use according to Claim 16 or 17, characterised in that the keto compound is used as the ion-selective component of an ion-selective unit for the determination of the concentration of the anions of oxy acids, for example as the ion-selective component of a membrane of an ion-selective electrode used for determining the concentration of anions, whereby the ion-selective unit may optionally contain as an additional component a quarternary ammonium compound with at least one lipophilic aliphatic hydrocarbon chain with at least eight carbon atoms.

19. Use according to one of Claims 16 to 18, characterised in that an azoketo compound of Formula II according to the definitions in one of Claims 3 or 5-7 is used.

20. Use according to Claim 16 or 17, characterised in that a keto compound according to the definitions in Claim 2 or 3 is used, in which the keto group is part of a chromophore or the keto compound has a fluorescing group, and whereby these keto compounds are used as colour indicators, in which the reaction of the oxy acid anion with the keto compound produces a shift in the visible or ultraviolet spectrum absorption or a fluorescence is either produced or extinguished.

21. Use according to Claim 16, characterised in that the keto compound is used as a component of a test device whereby the keto compound is present either on or in a carrier material, for example a plastic carrier or a carrier material of paper, whereby on contact with oxy acid anions a colour change appears in the visible or ultraviolet spectra or the test device is a sensor incorporating the keto compound and in which a colour change occurs on contact with oxy acid anions or a fluorescence appears or a fluorescence is extinguished and whereby the sensors are preferably the sensors of optodes in which the sensor can be introduced into regions that are accessible with difficulty and the change in colour or fluorescence on contact with the anions of oxy acids may be transmitted by a conducting device such as an optical fibre and thus observed.

22. Use according to Claim 20 or 21, characterised in that a keto compound according to the definition in one of the Claims 2 or 3 or 5 to 7 is used.

## Claims (Claims for the following Contracting State(s): ES)

1. Process for the preparation of adducts, chelates or complexes from anions and keto compounds, characterised in that anions of oxy acids are brought into contact with keto compounds which are monoketones of Formula I 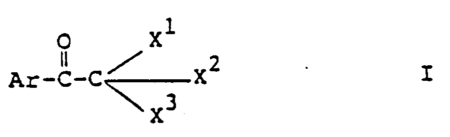 in which Ar is a substituted or unsubstituted mono- or polynuclear aromatic group or is a substituted or unsubstituted mono- or polynuclear heterocyclic group of aromatic character, preferably a benzene nucleus, naphthalene, anthracene, thiophene, furan, pyrrole, indole, benzofuran, benzothiophene, pyridine, quinoline, pyrazine or triazene, where the aromatic or heteroaromatic ring system may bear one or more of the following groups as substituents: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkyl-amino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups, and in which x¹, x², x³ may represent, independently of one another, hydrogen atoms, alkyl groups, alkenyl groups, alkinyl groups, fluorine atoms, chlorine atoms, bromine atoms or nitro groups, with the condition that at least one of the substituents x¹, x², x³ is a strongly electron-attracting substituent chosen from the selection: fluorine, chlorine or bromine atoms or nitro groups and with the further condition that when Ar is an alkoxycarbonylphenyl or alkylphenyl, the groups x¹, x², x³ may not all represent fluorine atoms.

2. Process according to Claim 1, characterised in that adducts are prepared with such keto compounds of Formula I in which the keto compounds possess a fluorescing group, such that the keto compounds are distinguished from their adduct with an oxy acid anion by aborption in the visible or ultraviolet spectrum or whereby upon adduct formation from the keto compound with an oxy acid anion a fluorescence is produced or extinguished.

3. Process according to Claim 1 or 2, characterised in that for preparation of the adducts keto compounds are used which are azo compounds of Formula II 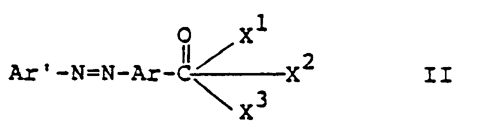 in which the radicals Ar and Ar' independently of each other represent substituted or unsubstituted mono- or polynuclear aromatic or substituted or unsubstituted mono- or polynuclear heterocyclic groups of aromatic character and x¹, x² and x³ represent independently of each other hydrogen atoms, alkyl groups, alkenyl groups, alkinyl groups, fluorine atoms, chlorine atoms, bromine atoms or nitro groups, with the provision that at least one of the substituents x¹, x² and x³ is a strongly electron-attracting substituent selected from the group: fluorine, chlorine or bromine atoms or nitro groups, and where the keto compounds of Formula II are distinguished from their adducts with oxy acid anions by absorption in the visible or ultraviolet spectrum.

4. Process according to one of Claims 1-3, characterised in that the oxy acid anions used to form the adduct, preferably a complex, with the keto compound are CO₃⁻⁻, HCO₃⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, IO₃⁻, NO₃⁻, NO₂⁻, CrO₄⁻⁻, HCrO₄⁻, Cr₂O₇⁻⁻, HCr₂O₇⁻ or the anions of carboxylic, hydroxycarboxylic, ketocarboxylic or aminocarboxylic acids or the anions of peptides, or the anions of organic derivatives of phosphoric acid, particularly esters of phosphoric, diphosphoric or triphosphoric acids, for example adenylic acid, adenosine diphosphate or adenosine triphosphate.

5. Process according to Claim 3 or 4, characterised in that an azo compound of Formula II is used in which the radicals Ar and Ar' denote independently of each other the aromatic rings or ring systems: benzene, naphthalene or anthracene or the heterocyclic radicals of aromatic character: thiophene, furan,pyrrole, indole, benzofuran, benzothiophene, pyridine, quinoline, pyrazine or triazine, whereby the said aromatic or heteroaromatic ring systems may bear one or more of the following groups as substituents: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkyl-amino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups.

6. Process according to one of the Claims 3 to 5, characterised in that an adduct of an azoketo compound of Formula III 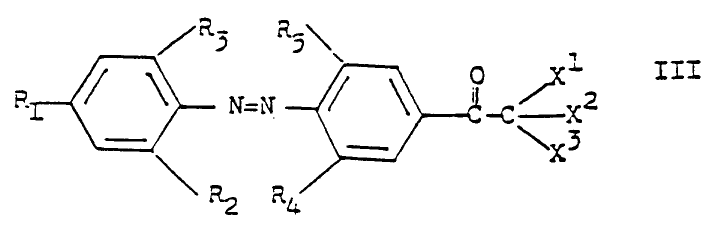 is prepared, in which the substituents R₁, R₂, R₃, R₄ and R₅ independently of each other represent hydrogen atoms, hydroxy groups, alkyl ether groups, aryl ether groups, esterified hydroxy groups, amino groups, alkylamino groups in which the alkyl radical may optionally be substituted, dialkylamino groups in which the alkyl groups may optionally be substituted, arylamino groups in which the aryl radical may optionally be substituted, nitro groups, optionally substituted alkyl groups, optionally substituted alkenyl groups, optionally substituted alkinyl groups or substituted or unsubstituted mono- or polynuclear aromatic or substituted or unsubstituted mono- or polynuclear heterocyclic radicals of aromatic character.

7. Process according to one of the Claims 1 to 6, characterised in that, for the preparation of the adducts, a keto component of Formulae I, II or III is used, in which in the group of the Formula 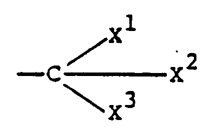 at least two of the radicals x¹, x² and x³ are strongly electron-attracting substituents chosen from the selection: fluorine, chlorine or bromine atoms or nitro groups, and furthermore in those compounds of Formula I in which Ar represents a grouping other than alkoxycarbonylphenyl or alkylphenyl, and in the compounds of Formulae II and III, preferably all of the radicals x¹, x² and x³ are fluorine atoms.

8. Process for the preparation of keto compounds of Formula I 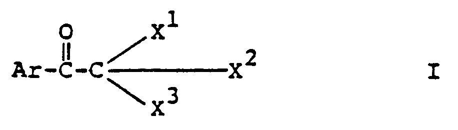 in which Ar is a substituted or unsubstituted mono- or polynuclear aromatic residue or is a substituted or unsubstituted mono- or polynuclear heterocyclic residue of aromatic character, preferably a benzene nucleus, naphthalene, anthracene, thiophene, furan, pyrrole, indole, benzofuran, benzothiophene, pyridine, quinoline, pyrazine or triazene, where the aromatic or heteroaromatic ring systems may bear one or more of the following groups as substituents: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkyl-amino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups, and in which x¹, x², x³ may represent, independently of one another, hydrogen atoms, alkyl groups, alkenyl groups, alkinyl groups, fluorine atoms, chlorine atoms, bromine atoms or nitro groups, with the condition that at least one of the substituents x¹, x², x³ is a strongly electron-attracting substituent chosen from the selection: fluorine, chlorine or bromine atoms or nitro groups and with the further condition that when Ar is an alkoxycarbonylphenyl or alkylphenyl, the groups x¹, x², x³ may not all represent fluorine atoms, characterised in that an aromatic or heteroaromatic compound of Formula IX
ArH IX
is reacted with an acyl halide of Formula X 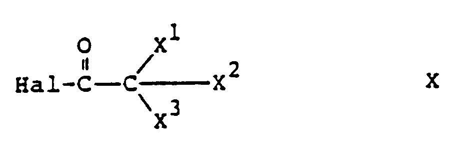 by the Friedel-Crafts acylation procedure and the keto compound of Formula I thus formed is isolated.

9. Process according to Claim 8, characterised in that an azoketo compound of Formula II is prepared 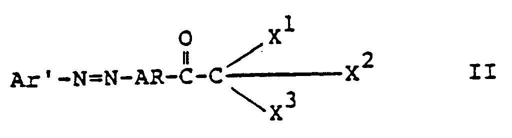 in which the radicals Ar and Ar' independently of each other represent substituted or unsubstituted mono- or polynuclear aromatic or substituted or unsubstituted mono- or polynuclear heterocyclic groups of aromatic character and x¹, x² and x³ represent independently of each other hydrogen atoms, alkyl groups, alkenyl groups, alkinyl groups, fluorine atoms, chlorine atoms, bromine atoms or nitro groups, with the provision that at least one of the substituents x¹, x² and x³ is a strongly electron-attracting substituent selected from the group fluorine, chlorine or bromine atoms or nitro groups, in which either a primary aromatic or heteroaromatic amine of Formula V 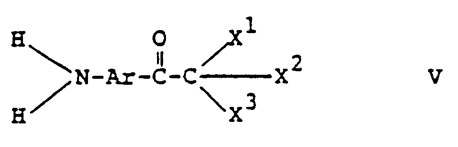 is diazotized and subjected to an azo coupling with an aromatic or heteroaromatic compound of Formula VI
Ar'-H VI
whereby the azo compound of Formula II is formed, or in which a primary aromatic or heteroaromatic amine of Formula VII 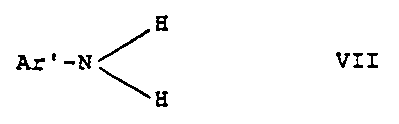 is diazotized and subjected to a azo coupling with an aromatic or heteroaromatic compound of Formula VIII 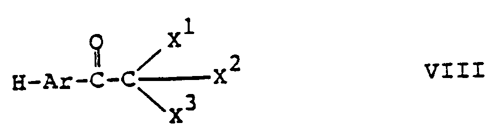 whereby the azo compound of Formula II is formed.

10. Process according to Claim 9, characterised in that azoketo compounds of Formula II are prepared in which the radicals Ar and Ar' independently of each other represent the aromatic ring systems benzene, naphthalene or anthracene or the heterocyclic residues of aromatic character: thiophene, furan, pyrrole, indole, benzofuran, benzothiophene, pyridine, quinoline, pyrazine or triazene, which may bear one or more substituents from the following group: halogen atoms, nitro groups, alkyl groups, azo groups, halogen-substituted alkyl groups, ether groupings, carboxylic ester groupings, hydroxy groups, esterified hydroxy groups, amino groups, alkylamino groups in which the alkyl residue may optionally bear substituents, dialkylamino groups in which the alkyl residues may optionally bear substituents, arylamino or diarylamino groups in which the aryl residues may optionally bear substituents, cycloalkyl- or dicycloalkyl-amino groups in which the cycloalkyl groups may optionally bear substituents, substituted alkyl groups, substituted or unsubstituted alkenyl groups, substituted or unsubstituted alkinyl groups, substituted or unsubstituted cycloalkyl groups and substituted or unsubstituted aryl groups.

11. Process according to Claim 10, characterised in that azoketo compounds of Formula III 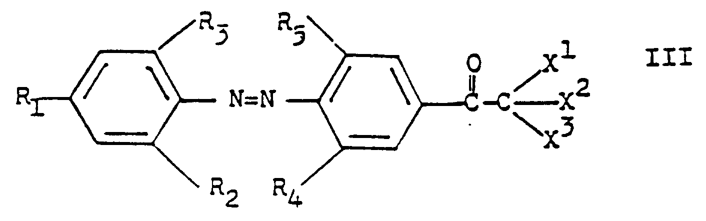 are prepared, in which the substituents R₁, R₂, R₃, R₄ and R₅ independently of each other represent hydrogen atoms, hydroxy groups, alkyl ether groups, aryl ether groups, esterified hydroxy groups, amino groups, alkylamino groups in which the alkyl radical may optionally be substituted, dialkylamino groups in which the alkyl groups may optionally be substituted, arylamino groups in which the aryl radical may optionally be substituted, nitro groups, optionally substituted alkyl groups, optionally substituted alkenyl groups, optionally substituted alkinyl groups or substituted or unsubstituted mono- or polynuclear aromatic or substituted or unsubstituted mono- or polynuclear heterocyclic radicals of aromatic character.

12. Process according to Claim 11, characterised in that azoketo compounds of Formula III are prepared, in which the radicals R₄ and R₅ are hydrogen atoms and one or two of the radicals R₁, R₂ and R₃ represent a hydroxy group, an esterified hydroxy group, a nitro group, an amino group, a monoalkylamino group with an optionally substituted alkyl group, a dialkylamino group with an optionally substituted alkyl group or an aryl- or diarylamino group with an optionally substituted aryl radical or radicals and where the radical R₁ is preferably one of the substituents mentioned and the radicals R₂ and R₃ represent hydrogen atoms.

13. Use of keto compounds of Formula I 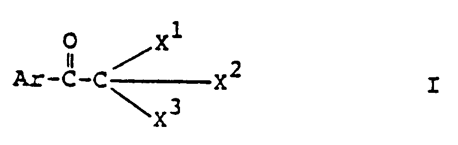 in which Ar, x¹, x² and x³ have the same significance as in Claim 1 for the detection of oxy acid anions, in that the keto compounds of Formula I are brought into contact with the anions to be detected, with the formation of the adducts defined in Claim 1.

14. Use according to Claim 13, characterised in that the oxy acid anions used are CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, IO₃⁻, NO₃⁻, NO₂⁻, CrO₄⁻⁻, HCrO₄⁻, Cr₂O₇⁻⁻, HCr₂O₇⁻ or the anions of carboxylic, hydroxycarboxylic, ketocarboxylic, aminocarboxylic acids or the anions of peptides, or the anions of organic derivatives of phosphoric acid, particularly esters of phosphoric, diphosphoric or triphosphoric acids, for example adenylic acid, adenosine diphosphate or adenosine triphosphate.

15. Use according to Claim 13 or 14, characterised in that the keto compound is used as the ion-selective component of an ion-selective unit for the determination of the concentration of the anions of oxy acids, for example as the ion-selective component of a membrane of an ion-selective electrode used for determining the concentration of anions, whereby the ion-selective unit may optionally contain a quarternary ammonium compound with at least one lipophilic aliphatic hydrocarbon chain with at least eight carbon atoms.

16. Use according to one of the Claims 13 to 15, characterised in that an asoketo compound of Formula II according to the definition given in one of the Claims 3 or 5-7 is used.

17. Use according to Claims 13 or 14, characterised in that a keto compound according to the definition given in Claim 2 or 3 is used, in which the keto group is part of a chromophore or the keto compound has a fluorescing group, and whereby these keto compounds are used as colour indicators, in which reaction of the oxy acid anion with the keto compound produces a shift in the visible or ultraviolet spectrum absorption or a fluorescence is either produced or extinguished.

18. Use according to Claim 13, characterised in that the keto compound is used as a component of a test device whereby the keto compound is present either on or in a carrier material, for example a plastic carrier or a carrier material of paper, or whereby the test device is a sensor which incorporates the keto compound and displays, on contact with oxy acid anions, a colour change in the visible or ultraviolet spectrum or a fluorescence appears or a fluorescence is extinguished, and whereby the sensors are preferably the sensors of optodes in which the sensor can be introduced into regions that are difficult of access and the change in colour or fluorescence on contact with the oxy acid anions may be transmitted by a conducting device such as an optical fibre and thus observed.

19. Use according to Claim 17 or 18, characterised in that a keto compound according to the definition given in one of Claims 2 or 3 or 5-7 is used.

## Revendications (Revendications pour l'(les) Etat(s) contractant(s) suivant(s): AT, BE, CH, DE, FR, GB, IT, LI, LU, NL, SE)

1. Adduits d'anions et de cétocomposés, caractérisés en ce que les anions sont des anions d'oxacides et en ce que les cétocomposés sont des monocétones de formule I 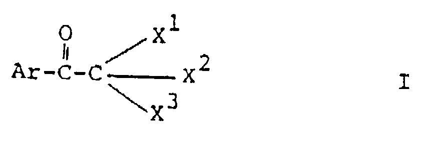 dans laquelle
Ar désigne un radical aromatique substitué ou non substitue, à un ou plusieurs noyaux ou un radical hétérocyclique à caractère aromatique substitué ou non substitué, à un ou plusieurs noyaux, de préférence un noyau benzène, un groupe naphtalène, anthracène, thiophène, furane, pyrrole, indole, benzofurane, benzothiophène, pyridine, quinoléine, pyrazine ou triazine, les systèmes cycliques aromatiques ou hétéroaromatiques pouvant porter comme substituants un ou plusieurs des groupes suivants: atomes d'halogène, groupes nitro, alkyle, azo, groupes alkyle halogénosubstitués, groupes éther, groupes esters d'acide carboxylique, groupes hydroxy, groupes hydroxy estérifiés, groupes amino, groupes alkylamino, dans lesquels le radical alkyle porte éventuellement des substituants, groupes dialkylamino, dans lesquels les radicaux alkyle portent éventuellement des substituants, groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués et
X₁, X₂ et X₃ désignent indépendamment l'un de l'autre des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons, choisi dans le groupe des atomes de fluor, de chlore, de brome ou des groupes nitro et à condition encore que, lorsque Ar désigne un radical alcoxycarbonylphényle ou alkylphényle, tous les radicaux X₁, X₂ et X₃ ne doivent pas désigner des atomes de fluor.

2. Adduits selon la revendication 1, caractérisés en ce que les cétocomposés de formule I sont ceux dans lesquels le groupe céto est une partie d'un chromophore et en ce que les cétocomposés présentent un groupe fluorescent, ces cétocomposés se distinguant de l'adduit formé par lui-même et un anion d'un oxacide quant à son absorption dans la gamme visible ou dans la gamme ultraviolette et la formation d'adduits du cétocomposé avec l'anion de l'oxacide provoquant l'apparition ou la disparition d'une fluorescence.

3. Adduits selon la revendication 1 ou 2, caractérisés en ce que leurs cétocomposés sont des composés azoïques de formule II 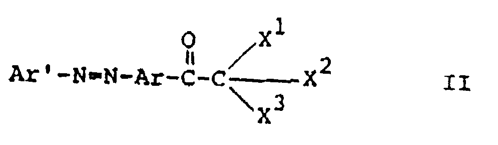 dans laquelle les radicaux
Ar et Ar' désignent, indépendamment l'un de l'autre, des radicaux aromatiques substitués ou non substitués, à un ou plusieurs noyaux ou des radicaux hétérocycliques à caractère aromatique substitués ou non substitués à un ou plusieurs noyaux et
X₁, X₂ et X₃ désignent, indépendamment l'un de l'autre, des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons, choisi parmi le groupe des atomes de fluor, des atomes de chlore, de brome ou des groupes nitro,
les cétocomposés de formule II se distinguant des adduits de ces derniers avec les anions d'oxacides quant à l'absorption dans la gamme visible ou dans la gamme ultraviolette.

4. Adduits selon une des revendications 1 à 3, caractérisés en ce que l'anion de l'oxacide qui forme l'adduit, de préférence un complexe, avec le cétocomposé, est CO₃⁻⁻, HCO₃⁻⁻, SO₄⁻⁻, HSO₄⁻, SO₃⁻⁻, HSO₃⁻, PO₄⁻⁻⁻, HPO₄⁻⁻, H₂PO₄⁻, Cl0₃⁻, Cl0₄⁻, BrO₃⁻, IO₃⁻, NO₃⁻, NO₂⁻, CrO₄⁻⁻, HCrO₄⁻, Cr₂O₇⁻⁻, HCr₂O₇⁻ ou un anion d'un acide carboxylique, d'un acide hydroxycarboxylique, d'un acide cétocarboxylique ou d'un acide aminocarboxylique, d'un peptide ou d'un dérivé organique de l'acide phosphorique, en particulier d'un ester de l'acide phosphorique, de l'acide diphosphorique ou de l'acide triphosphorique, par exemple de l'acide adénylique, de l'adénosinediphosphate ou de l'adénosinetriphosphate.

5. Adduits selon la revendication 3 ou 4, caractérisés en ce que, dans le composé azoïque de formule II, les radicaux
Ar et Ar' désignent, indépendamment l'un de l'autre, les systèmes cycliques aromatiques benzène, naphtalène ou anthracène ou les radicaux hétérocycliques de caractère aromatique thiophène, furane, pyrrole, indole, benzofurane, benzothiophène, pyridine, quinoléine, pyrazine ou triazine, les systèmes cycliques aromatiques ou hétéroaromatiques cités pouvant porter comme substituants un ou plusieurs des groupes suivants: des atomes d'halogène, des groupes nitro, des groupes alkyle, des groupes azo, des groupes alkyle halogénosubstitués, des groupes éther, des groupes ester d'acide carboxylique, des groupes hydroxy, des groupes hydroxy estérifiés, des groupes amino, des groupes alkylamino, dans lesquels le radical alkyle porte éventuellement des substituants, des groupes dialkylamino, dans lesquels les radicaux alkyle portent éventuellement des substituants, des groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués.

6. Adduits selon une des revendications 3 à 5 caractérisés en ce que l'azocétocomposé de l'adduit répond à la formule III 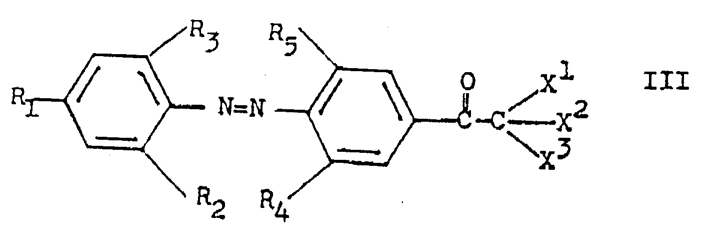 dans laquelle les substituants
R₁, R₂, R₃, R₄ et R₅ désignent, indépendamment l'un de l'autre, des atomes d'hydrogène, des groupes hydroxy, des groupes alkyléther, des groupes aryléther, des groupes hydroxy estérifiés, des groupes amino, des groupes alkylamino dont le radical alkyle est éventuellement substitué, des groupes dialkylamino dont les groupes alkyle sont éventuellement substitués, des groupes arylamino dont le radical aryle est éventuellement substitué, des groupes nitro, des groupes alkyle éventuellement substitués, des groupes alcényle éventuellement substitués, des groupes alcynyle éventuellement substitués ou des radicaux aromatiques substitués ou non substitués à un ou plusieurs noyaux ou des radicaux hétérocycliques à caractère aromatique substitués ou non substitués à un ou plusieurs noyaux.

7. Adduits selon une des revendications 1 à 6 caractérisés en ce que, dans les cétocomposés de formules I, II ou III des adduits, au moins deux des radicaux X₁, X₂ et X₃ désignent des substituants fortement attracteurs d'électrons, choisis dans le groupe des atomes de fluor, de chlore, de brome ou des groupes nitro dans le groupe de formule 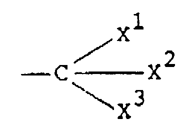 tous les radicaux X₁, X₂ et X₃ désignant de préférence des atomes de fluor dans les composés de formule I dans laquelle Ar possède une autre signification qu'alcoxycarbonylphényle ou alkylphényle et dans les composés de formules II et III.

8. Procédé de préparation d'un adduit à partir d'anions et de cétocomposés selon la revendication 1, caractérisé en ce que l'anion d'un oxacide entre en contact avec le cétocomposé, l'adduit se formant à cette occasion.

9. Procédé selon la revendication 8, caractérisé en ce qu'un adduit selon une des revendications 2 à 7 est préparé.

10. Cétocomposés pour l'exécution du procédé selon la revendication 8, caractérisés en ce que ce sont des azocétocomposés de formule II 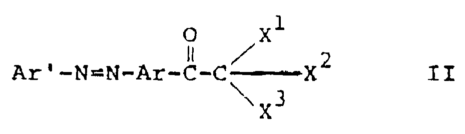 dans laquelle les radicaux
Ar et Ar' désignent, indépendamment l'un de l'autre, des radicaux aromatiques substitués ou non substitués à un ou plusieurs noyaux ou des radicaux hétérocycliques à caractère aromatique substitués ou non substitués à un ou plusieurs noyaux et
X₁, X₂ et X₃ désignent, indépendamment l'un de l'autre, des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons choisi dans le groupe des atomes de fluor, de chlore, de brome ou des groupes nitro, au moins deux des substituants X₁, X₂ et X₃ étant de tels substituants fortement attracteurs d'électrons et, tout particulièrement, tous les substituants X₁, X₂ et X₂ désignant des atomes de fluor dans les azocétocomposés de formule II.

11. Azocétocomposés selon la revendication 10 caractérisés en ce que, dans les composés de formule II, les radicaux
Ar et Ar' désignent, indépendamment l'un de l'autre, les systèmes cycliques aromatiques benzène, naphtalène ou anthracène ou les radicaux hétérocycliques à caractère aromatique thiophène, furane, pyrrole, indole, benzofurane, benzothiophène, pyridine, quinoléine, pyrazine ou triazine, qui peuvent porter un ou plusieurs substituants choisis parmi le groupe suivant:
des atomes d'halogène, des groupes nitro, des groupes alkyle, des groupes azo, des groupes alkyle halogénosubstitués, des groupes éther, des groupes ester d'acide carboxylique, des groupes hydroxy, des groupes hydroxy estérifiés, des groupes amino, des groupes alkylamino, dans lesquels le radical alkyle porte éventuellement des substituants, des groupes dialkylamino, dans lesquels les radicaux alkyle portent éventuellement des substituants, des groupes arylamino ou diarylamino dans lesquels les groupes aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués.

12. Azocétocomposés selon la revendication 11 caractérisés par la formule III 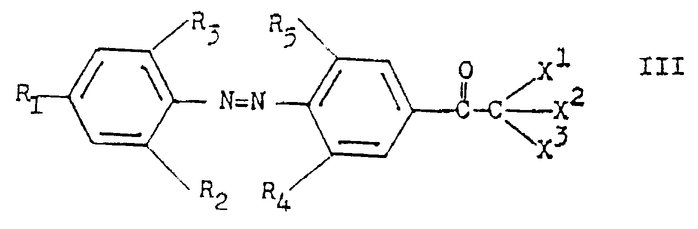 dans laquelle les substituants
R₁, R₂, R₃, R₄ et R₅ désignent, indépendamment l'un de l'autre, des atomes d'hydrogène, des groupes hydroxy, des groupes alkyléther, des groupes aryléther, des groupes hydroxy estérifiés, des groupes amino, des groupes alkylamino dont le radical alkyle est éventuellement substitué, des groupes dialkylamino dont les groupes alkyle sont éventuellement substitués, des groupes arylamino dont le radical aryle est éventuellement substitué, des groupes nitro, des groupes alkyle éventuellement substitués, des groupes alcényle éventuellement substitués, des groupes alcynyle éventuellement substitués ou des radicaux aromatiques substitués ou non substitués à un ou plusieurs noyaux ou des radicaux hétérocycliques à caractère aromatique substitués ou non substitués à un ou plusieurs noyaux.

13. Azocétocomposés selon la revendication 12, caractérisés en ce que, chez eux, les radicaux R₄ et R₅ désignent des atomes d'hydrogène et
un ou deux des radicaux R₁, R₂ et R₃ désignent un groupe hydroxy, un groupe hydroxy estérifié, un groupe nitro, un groupe amino, un groupe monoalkylamino avec un groupe alkyle éventuellement substitué, un groupe dialkylamino à groupe alkyle éventuellement substitué ou un groupe
arylamino ou diarylamino, le ou les radicaux aryle portant éventuellement des substituants et le radical R₁ étant de préférence un des substituants précités tandis que les radicaux R₂ et R₃ désignent des atomes d'hydrogène.

14. Procédé de préparation de nouveaux azocétocomposés selon la revendication 10 caractérisé en ce que sont préparés des azocétocomposés de formule II 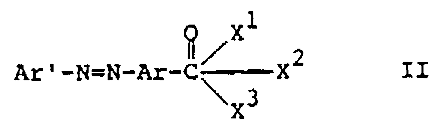 dans laquelle les radicaux
Ar, Ar', X₁, X₂ et X₃ possèdent la signification spécifiée à la revendication 10, soit en diazotant une amine aromatique ou hétéroaromatique primaire de formule V 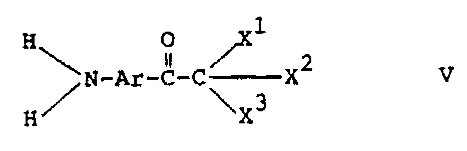 et en la soumettant à une copulation azoïque avec un compose aromatique ou hétéroaromatique de formule VI
Ar'-H VI
l'azocomposé de formule II étant ainsi formé,
ou en diazotant une amine aromatique ou hétéroaromatique primaire de formule VII 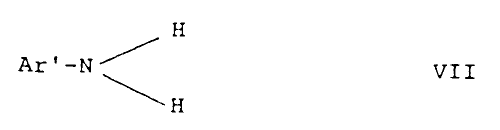 et en la soumettant à une copulation azoïque avec un composé aromatique ou hétéroaromatique de formule VIII, 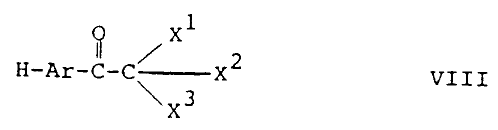 l'azocomposé de formule II étant ainsi formé.

15. Procédé selon la revendication 14, caractérisé en ce que les azocétocomposés définis dans les revendications 11 à 13 sont préparés.

16. Mise en oeuvre des cétocomposés de formule I indiquée dans la revendication 1 pour détecter des anions d'oxacides en mettant en contact les cétocomposés de formule I avec les anions à détecter, en formant les adduits définis à la revendication 1.

17. Mise en oeuvre selon la revendication 16, caractérisée en ce que les anions d'oxacides mis en oeuvre sont CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, ClO₄⁻, BrO₃⁻, IO₃⁻, NO₃⁻, NO₂⁻, CrO₄⁻⁻, HCrO₄⁻, Cr₂O₇⁻⁻, HCr₂O₇⁻ ou des anions d'acides carboxyliques, d'acides hydroxycarboxyliques, d'acides cétocarboxyliques ou d'acides aminocarboxyliques, de peptides ou de dérivés organiques de l'acide phosphorique, en particulier d'esters de l'acide phosphorique, de l'acide diphosphorique ou de l'acide triphosphorique, par exemple de l'acide adénylique, de l'adénosinediphosphate ou de l'adénosinetriphosphate.

18. Mise en oeuvre selon la revendication 16 ou 17 caractérisée en ce que le cétocomposé est utilisé comme composant sélecteur d'ions d'une partie sélectrice d'ions pour la détermination de la concentration des anions d'oxacides, par exemple comme composant sélecteur d'ions d'une membrane d'une électrode sélectrice d'ions pour la détermination de la concentration des anions, la partie sélectrice d'ions contenant éventuellement comme autre composant un composé d'ammonium quaternaire avec au moins une chaîne hydrocarbure aliphatique lipophile avec au moins huit atomes de carbone.

19. Mise en oeuvre selon une des revendications 16 à 18, caractérisée en qu'un azocétocomposé de formule II selon la définition donnée dans une des revendications 3 ou 5 - 7 est mis en oeuvre.

20. Mise en oeuvre selon la revendication 16 ou 17 caractérisée en ce qu'est mis en oeuvre un cétocomposé selon la définition donnée dans la revendication 2 ou 3 dans laquelle le groupe céto est une partie d'un chromophore ou le cétocomposé présente un groupe fluorescent, ces cétocomposés étant mis en oeuvre comme indicateurs colorés, auquel cas une variation de l'absorption du cétocomposé dans la gamme visible ou ultraviolette ou l'apparition ou la disparition d'une fluorescence se produit par la réaction de l'anion des oxacides avec le cétocomposé.

21. Mise en oeuvre selon la revendication 16, caractérisée en ce que le cétocomposé est mis en oeuvre comme composant d'un dispositif de test, le cétocomposé se trouvant sur ou dans un matériau de support, par exemple dans un matériau de support en plastique ou en papier ou le dispositif de test étant un détecteur qui contient le cétocomposé et qui montre un virage de couleur dans la gamme visible ou ultraviolette ou l'apparition ou la disparition d'une fluorescence en cas de contact avec des anions d'oxacides et les détecteurs étant de préférence les détecteurs d'optodes, auquel cas le détecteur peut être introduit dans des zones difficilement accessibles et la variation de la couleur ou de la fluorescence est observée par un transmetteur, par exemple un guide d'ondes, en cas de contact avec les anions d'oxacides.

22. Mise en oeuvre selon la revendication 20 ou 21 caractérisée en ce qu'un cétocomposé selon la définition donnée aux revendications 2, 3 ou 5 à 7 est mis en oeuvre.

## Revendications (Revendications pour l'(les) Etat(s) contractant(s) suivant(s): ES)

1. Procédé de préparation d'adduits, de chélates, ou de complexes d'anions et de cétocomposés, caractérisé en ce que des anions d'oxacides sont mis en contact avec des cétocomposés, ces cétocomposés étant des monocétones de formule I 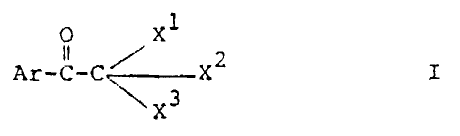 dans laquelle
Ar désigne un radical aromatique substitué ou non substitué, à un ou plusieurs noyaux ou un radical hétérocyclique à caractère aromatique substitué ou non substitué, à un ou plusieurs noyaux, de préférence un noyau benzène, un groupe naphtalène, anthracène, thiophène, furane, pyrrole, indole, benzofurane, benzothiophène, pyridine, quinoléine, pyrazine ou triazine, les systèmes cycliques aromatiques ou hétéroaromatiques pouvant porter comme substituants un ou plusieurs des groupes suivants: atomes d'halogène, groupes nitro, alkyle, azo, groupes alkyle halogénosubstitués, groupes éther, groupes esters d'acide carboxylique, groupes hydroxy, groupes hydroxy estérifiés, groupes amino, groupes alkylamino, dans lesquels le radical alkyle porte éventuellement des substituants, groupes dialkylamino, dans lesquels les radicaux alkyle portent éventuellement des substituants, groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués et
X₁, X₂ et X₃ désignent indépendamment l'un de l'autre des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons, choisi dans le groupe des atomes de fluor, de chlore, de brome ou des groupes nitro et à condition encore que, lorsque Ar désigne un radical alcoxycarbonylphényle ou alkylphényle, tous les radicaux X₁, X₂ et X₃ ne doivent pas désigner des atomes de fluor.

2. Procédé selon la revendication 1, caractérisé en ce que des adduits sont préparés avec de tels cétocomposés de formule I, dans laquelle les cétocomposés présentent un groupe fluorescent, ces cétocomposés se distinguant de l'adduit formé par lui-même et un anion d'un oxacide quant à son absorption dans la gamme visible ou dans la gamme ultraviolette et la formation d'adduits du cétocomposé avec l'anion de l'oxacide provoquant l'apparition ou la disparition d'une fluorescence.

3. Procédé selon la revendication 1 ou 2, caractérisé en ce que la préparation des adduits met en oeuvre des cétocomposés qui sont des composés azoïques de formule II 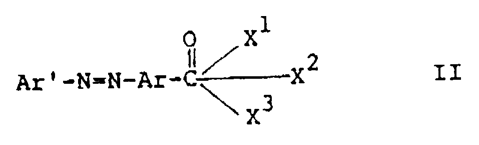 dans laquelle les radicaux
Ar et Ar' désignent, indépendamment l'un de l'autre, des radicaux aromatiques substitués ou non substitués, à un ou plusieurs noyaux ou des radicaux hétérocycliques à caractère aromatique substitués ou non substitués à un ou plusieurs noyaux et
X₁, X₂ et X₃ désignent, indépendamment l'un de l'autre, des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons, choisi parmi le groupe des atomes de fluor, des atomes de chlore, de brome ou des groupes nitro,
les cétocomposés de formule II se distinguant des adduits de ces derniers avec les anions d'oxacides quant à l'absorption dans la gamme visible ou dans la gamme ultraviolette.

4. Procédé selon une des revendications 1 à 3, caractérisé en ce que l'anion de l'oxacide qui forme l'adduit, de préférence un complexe, avec le cétocomposé, est CO₃⁻⁻, HCO₃⁻⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, Cl0₄⁻, BrO₃⁻, IO₃⁻, NO₃⁻,
NO₂⁻, CrO₄⁻⁻, HCrO₄⁻, Cr₂O₇⁻⁻, HCr₂O₇⁻ ou un anion d'un acide carboxylique, d'un acide hydroxycarboxylique, d'un acide cétocarboxylique ou d'un acide aminocarboxylique, d'un peptide ou d'un dérivé organique de l'acide phosphorique, en particulier d'un ester de l'acide phosphorique, de l'acide diphosphorique ou de l'acide triphosphorique, par exemple de l'acide adénylique, de l'adénosinediphosphate ou de l'adénosinetriphosphate.

5. Procédé selon la revendication 3 ou 4, caractérisé en ce qu'est mis en oeuvre un composé azoïque de formule II dans laquelle les radicaux
Ar et Ar' désignent, indépendamment l'un de l'autre, les systèmes cycliques aromatiques benzène, naphtalène ou anthracène ou les radicaux hétérocycliques de caractère aromatique thiophène, furane, pyrrole, indole, benzofurane, benzothiophène, pyridine, quinoléine, pyrazine ou triazine, les systèmes cycliques aromatiques ou hétéroaromatiques cités pouvant porter comme substituants un ou plusieurs des groupes suivants: des atomes d'halogène, des groupes nitro, des groupes alkyle, des groupes azo, des groupes alkyle halogénosubstitués, des groupes éther, des groupes ester d'acide carboxylique, des groupes hydroxy, des groupes hydroxy estérifiés, des groupes amino, des groupes alkylamino, dans lesquels le radical alkyle porte éventuellement des substituants, des groupes dialkylamino, dans lesquels les radicaux alkyle portent éventuellement des substituants, des groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués.

6. Procédé selon une des revendications 3 à 5 caractérisé en ce qu'un adduit est préparé avec un azocétocomposé de formule III 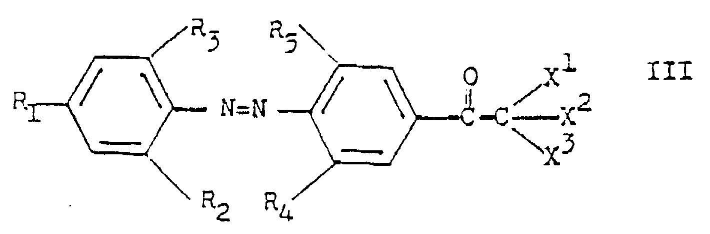 dans laquelle les substituants
R₁, R₂, R₃, R₄ et R₅ désignent, indépendamment l'un de l'autre, des atomes d'hydrogène, des groupes hydroxy, des groupes alkyléther, des groupes aryléther, des groupes hydroxy estérifiés, des groupes amino, des groupes alkylamino dont le radical alkyle est éventuellement substitué, des groupes dialkylamino dont les groupes alkyle sont éventuellement substitués, des groupes arylamino dont le radical aryle est éventuellement substitué, des groupes nitro, des groupes alkyle éventuellement substitués, des groupes alcényle éventuellement substitués, des groupes alcynyle éventuellement substitués ou des radicaux aromatiques substitués ou non substitués à un ou plusieurs noyaux ou des radicaux hétérocycliques à caractère aromatiques substitués ou non substitués à un ou plusieurs noyaux.

7. Procédé selon une des revendications 1 à 6 caractérisé en ce que la préparation des adduits met en oeuvre un cétocomposé de formules I, II ou III, au moins deux des radicaux X₁, X₂ et X₃ désignent des substituants fortement attracteurs d'électrons, choisis dans le groupe des atomes de fluor, de chlore, de brome ou des groupes nitro dans le groupe de formule 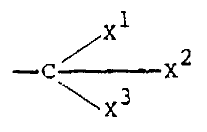 tous les radicaux X₁, X₂ et X₃ désignant de préférence des atomes de fluor dans les composés de formule I dans laquelle Ar possède une autre signification qu'alcoxycarbonylphényle ou alkylphényle et dans les composés de formules II et III.

8. Procédé de préparation de cétocomposés de formule I 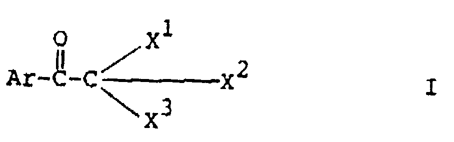 dans laquelle
Ar désigne un radical aromatique substitué ou non substitué à un ou plusieurs noyaux ou un radical hétérocyclique à caractère aromatique substitué ou non substitué à un ou plusieurs noyaux, de préférence un noyau benzène, un groupe naphtalène, anthracène, thiophène, furane, pyrrole, indole, benzofurane, benzothiophène, pyridine, quinoléine, pyrazine ou triazine, les systèmes cycliques aromatiques ou hétéroaromatiques pouvant contenir comme substituants un ou
plusieurs des groupes suivants: des atomes d'halogène, des groupes nitro, des groupes alkyle, des groupes azo, des groupes alkyle halogénosubstitués, des groupes éther, des groupes ester d'acide carboxylique, des groupes hydroxy, des groupes hydroxy estérifiés, des groupes amino, des groupes alkylamino, dans lesquels le radical alkyle porte éventuellement des substituants, des groupes dialkylamino, dans lesquels les radicaux alkyle portent éventuellement des substituants, des groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués et
X₁, X₂ et X₃ désignent, indépendamment l'un de l'autre, des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons choisi dans le groupe des atomes de fluor, de chlore, de brome ou des groupes nitro, et à condition encore que, lorsque Ar désigne un groupe alcoxycarbonylphényle ou alkylphényle, tous les radicaux X₁, X₂ et X₃ ne doivent pas désigner des atomes de fluor,
caractérisé en ce qu'un composé aromatique ou hétéroaromatique de formule IX
ArH IX
est amené à réagir avec un halogénure d'acyle de formule X 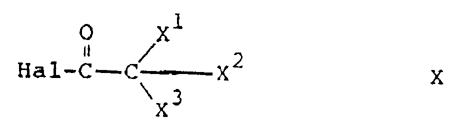 selon le procédé d'acylation de Friedel-Crafts et les cétocomposés formés de formule I sont isolés.

9. Procédé selon la revendication 8 caractérisé en ce que sont préparés des azocétocomposés de formule II 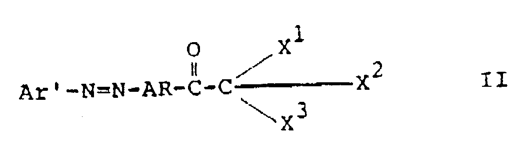 dans laquelle les radicaux
Ar et Ar' désignent, indépendamment l'un de l'autre, des radicaux aromatiques substitués ou non substitués à un ou plusieurs noyaux ou des radicaux hétérocycliques à caractère aromatique substitués ou non substitués à un ou plusieurs noyaux et
X₁, X₂ et X₃ désignent, indépendamment l'un de l'autre, des atomes d'hydrogène, des groupes alkyle, des groupes alcényle, des groupes alcynyle, des atomes de fluor, de chlore, de brome ou des groupes nitro, à condition toutefois qu'au moins un des substituants X₁, X₂ et X₃ désigne un substituant fortement attracteur d'électrons choisi dans le groupe des atomes de fluor, de chlore, de brome ou des groupes nitro, soit en diazotant une amine aromatique ou hétéroaromatique primaire de formule V 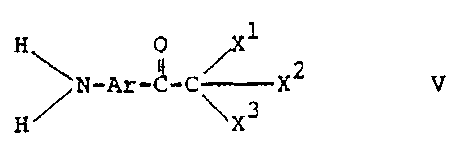 et en la soumettant à une copulation azoïque avec un composé aromatique ou hétéroaromatique de formule VI
Ar'-H VI
l'azocomposé de formule II étant ainsi formé,
ou en diazotant une amine aromatique ou hétéroaromatique primaire de formule VII 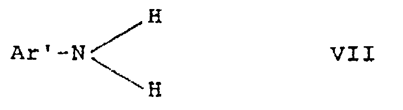 et en la soumettant à une copulation azoïque avec un composé aromatique ou hétéroaromatique de formule VIII, 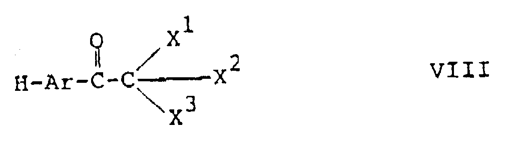 l'azocomposé de formule II étant ainsi formé.

10. Procédé selon la revendication 9, caractérisé en ce que sont préparés des azocétocomposés de formule II dans laquelle les radicaux
Ar et Ar' désignent, indépendamment l'un de l'autre, les systèmes cycliques aromatiques benzène, naphtalène, anthracène ou les radicaux hétérocycliques de caractère aromatique thiophène, furane, pyrrole, indole, benzofurane, benzothiophène, pyridine, quinoléine, pyrazine ou triazine, qui peuvent porter comme substituants un ou plusieurs des groupes suivants: des atomes d'halogène, des groupes nitro, des groupes alkyle, des groupes azo, des groupes alkyle halogénosubstitués, des groupes éther, des groupes ester d'acide carboxylique, des groupes hydroxy, des groupes hydroxy estérifiés, des groupes amino, des groupes alkylamino, dans lesquels le radical alkyle porte éventuellement des substituants, des groupes dialkylamino, dans lesquels les radicaux alkyle portent éventuellement des substituants, des groupes arylamino ou diarylamino dans lesquels les radicaux aryle portent éventuellement des substituants, des groupes cycloalkylamino ou dicycloalkylamino dans lesquels les groupes cycloalkyle portent éventuellement des substituants, des groupes alkyle substitués, des groupes alcényle substitués ou non substitués, des groupes alcynyle substitués ou non substitués, des groupes cycloalkyle substitués ou non substitués et des groupes aryle substitués ou non substitués.

11. Procédé selon la revendication 10, caractérisé en ce que sont préparés des azocétocomposés de formule III 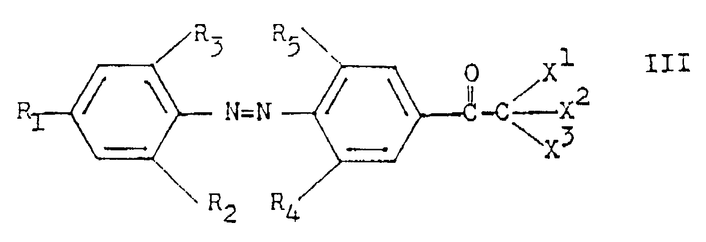 dans lesquels les substituants
R₁, R₂, R₃, R₄ et R₅ désignent, indépendamment l'un de l'autre, des atomes d'hydrogène, des groupes hydroxy, des groupes alkyléther, des groupes aryléther, des groupes hydroxy estérifiés, des groupes amino, des groupes alkylamino dont le radical alkyle est éventuellement substitué, des groupes dialkylamino dont les groupes alkyle sont éventuellement substitués, des groupes arylamino dont le radical aryle est éventuellement substitué, des groupes nitro, des groupes alkyle éventuellement substitués, des groupes alcényle éventuellement substitués, des groupes alcynyle éventuellement substitués ou des radicaux aromatiques substitués ou non substitués à un ou plusieurs noyaux ou des radicaux hétérocycliques à caractère aromatique substitués ou non substitués à un ou plusieurs noyaux.

12. Procédé selon la revendication 11, caractérisé en ce que sont préparés des azocétocomposés de formule III dans laquelle les radicaux
R₄ et R₅ sont des atomes d'hydrogène et
un ou deux des radicaux R₁, R₂ et R₃ désignent un groupe hydroxy, un groupe hydroxy estérifié, un groupe nitro, un groupe amino, un groupe monoalkylamino, avec un groupe alkyle éventuellement substitué, un groupe dialkylamino avec un groupe alkyle éventuellement substitué ou un groupe diarylamino, le ou les radicaux aryle portant éventuellement des substituants et le radical R₁ étant de préférence un des substituants cités tandis que les radicaux R₂ et R3 désignent des atomes d'hydrogène.

13. Mise en oeuvre de cétocomposés de formule I 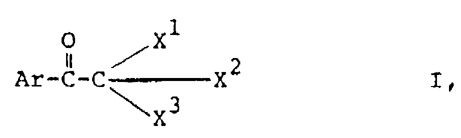 dans laquelle
Ar, X₁, X₂ et X₃ ont la même signification que dans la revendication 1, pour la détection d'anions d'oxacides en mettant en contact les cétocomposés de formule I avec les anions à détecter par formation des adduits définis à la revendication 1.

14. Mise en oeuvre selon la revendication 13, caractérisée en ce que les anions d'oxacides mis en oeuvre sont CO₃⁻⁻, HCO₂⁻, SO₄⁻⁻, HSO₄⁻, SO₃⁻⁻, HSO₃⁻, PO₄⁻⁻⁻, HPO₄⁻⁻, H₂PO₄⁻, ClO₃⁻, Cl0₄⁻, BrO₃⁻, IO₃⁻, NO₃⁻, NO₂⁻, CrO₄⁻⁻, HCrO₄⁻, Cr₂O₇⁻⁻, HCr₂O₇⁻ ou des anions d'acides carboxyliques, d'acides hydroxycarboxyliques, d'acides cétocarboxyliques ou d'acides aminocarboxyliques, de peptides ou de dérivés organiques de l'acide phosphorique, en particulier d'esters de l'acide phosphorique, de l'acide diphosphorique ou de l'acide triphosphorique, par exemple de l'acide adénylique, de l'adénosinediphosphate ou de l'adénosinetriphosphate.

15. Mise en oeuvre selon la revendication 13 ou 14 caractérisée en ce que le cétocomposé est utilisé comme composant sélecteur d'ions d'une partie sélectrice d'ions pour la détermination de la concentration des anions d'oxacides, par exemple comme composant sélecteur d'ions d'une membrane d'une électrode sélectrice d'ions pour la détermination de la concentration des anions, la partie sélectrice d'ions contenant éventuellement comme autre composant un composé d'ammonium quaternaire avec au moins une chaîne hydrocarbure aliphatique lipophile avec au moins huit atomes de carbone.

16. Mise en oeuvre selon une des revendications 13 à 15, caractérisée en qu'un azocétocomposé de formule II selon la définition donnée dans une des revendications 3 ou 5 - 7 est mis en oeuvre.

17. Mise en oeuvre selon la revendication 13 ou 14 caractérisée en ce qu'est mis en oeuvre un cétocomposé selon la définition donnée dans la revendication 2 ou 3 dans laquelle le groupe céto est une partie d'un chromophore ou le cétocomposé présente un groupe fluorescent, ces cétocomposés étant mis en oeuvre comme indicateurs colorés, auquel cas une variation de l'absorption du cétocomposé dans la gamme visible ou ultraviolette ou l'apparition ou la disparition d'une fluorescence se produit par la réaction de l'anion des oxacides avec le cétocomposé.

18. Mise en oeuvre selon la revendication 13, caractérisée en ce que le cétocomposé est mis en oeuvre comme composant d'un dispositif de test, le cétocomposé se trouvant sur ou dans un matériau de support, par exemple dans un matériau de support en plastique ou en papier ou le dispositif de test étant un détecteur qui contient le cétocomposé et qui montre un virage de couleur dans la gamme visible ou ultraviolette ou l'apparition ou la disparition d'une fluorescence en cas de contact avec des anions d'oxacides et les détecteurs étant de préférence les détecteurs d'optodes, auquel cas le détecteur peut être introduit dans des zones difficilement accessibles et la variation de la couleur ou de la fluorescence est observée par un transmetteur, par exemple un guide d'ondes, en cas de contact avec les anions d'oxacides.

19. Mise en oeuvre selon la revendication 17 ou 18 caractérisée en ce qu'un cétocomposé selon la définition donnée aux revendications 2 ou 3 ou 5 à 7 est mis en oeuvre.
